# EUROPEAN PATENT APPLICATION

(11) **EP 3 284 819 A1**
(43) Date of publication of application: **21.02.2018**
(21) Application number: 16184334.7
(22) Date of filing: 16.08.2016
(51) Int. Cl.: C12N 5/0783, C07K 14/12

(54) **METHOD FOR THE GENERATION OF T CELL HYBRIDOMA FOR CLONING AND ANALYSIS OF RECEPTORS USING ENGINEERED MEASLES VIRUS FUSOGENIC GLYCOPROTEINS**

(71) Applicant: Max-Delbrück-Centrum für Molekulare Medizin (MDC), 13125 Berlin (DE); Humboldt Universität zu Berlin, 10099 Berlin (DE)
(72) Inventor: Bunse, Mario, 10961 Berlin (DE); Edes, Inan, 10365 Berlin (DE); Uckert, Wolfgang, 13125 Berlin (DE)
(74) Representative: Moré, Solveig Helga

(57) **Abstract**

The invention relates to the field of immunology and immunotherapy, in particular, to adoptive T cell therapy of cancer utilizing T cell receptor (TCR)-engineered T cells. The invention provides novel methods and tools for identification and cloning of TCR, which are also applicable for identification of other receptors such as B cell receptors or antibodies. The invention provides immortalized cell lines able to induce subset specific (e.g. CD4⁺ or CD8⁺ specific) hybridization during co-culture, e.g., with primary lymphocytes of mice or men. The immortalized cells are engineered to express two mutated glycoproteins derived from the paramyxovirus family, namely hemagglutinin (H) and fusion (F) or a derivative thereof. H is a chimeric protein not able to bind to its natural ligands, fused to a targeting ligand capable of specifically binding to a specific cell surface antigen such as a lymphocytic subset-marking ligand (e.g. CD4 or CD8); fusion (F) mediates the fusion of cellular membranes of the immortalized cell line and cells, e.g., primary lymphocytes, H has bound to. The methods of the invention comprise preparing a hybridoma cell expressing a receptor, comprising steps of co-culturing a cell expressing a specific cell surface antigen and said receptor with the specific immortalized cells of the invention. The invention also relates to the hybridoma cells obtainable by said method.

## Description

The invention relates to the field of immunology and immunotherapy, in particular, to adoptive T cell therapy of cancer utilizing T cell receptor (TCR)-engineered T cells. The invention provides novel methods and tools for identification and cloning of TCR, which are also applicable for identification of other receptors such as B cell receptors or antibodies. The invention provides immortalized cell lines able to induce subset specific (e.g. CD4⁺ or CD8⁺ specific) hybridization during co-culture, e.g., with primary lymphocytes of mice or men. The immortalized cells are engineered to express two mutated glycoproteins derived from the paramyxovirus family, namely the attachment protein, e.g., hemagglutinin (H) and fusion (F) or a derivative thereof. In the context of the invention, H is used as a chimeric protein not able to bind to its natural ligands, fused to a targeting ligand capable of specifically binding to a specific cell surface antigen such as a lymphocytic subset-marking ligand (e.g. CD4 or CD8). Fusion (F) mediates the fusion of cellular membranes of the immortalized cell line and cells, e.g., primary lymphocytes, H has bound to. The methods of the invention comprise preparing a hybridoma cell expressing a receptor, with steps of co-culturing a cell expressing a specific cell surface antigen and said receptor with the specific immortalized cells of the invention. The invention also relates to the hybridoma cells obtainable by said method.

T cell receptor (TCR) gene therapy is a promising immunotherapeutic strategy to treat a variety of virus- and cancer-related indications. One key obstacle is the lack of availability of potent TCRs to be analyzed in preclinical and clinical settings. Current methods to detect and isolate antigen-specific TCRs are time-consuming, laborious and require highly trained personal and/or high-tech equipment.

Recently, two immunotherapeutic strategies have yielded impressive clinical results treating end-stage cancer patients. First, the usage of checkpoint inhibitors, which enable tumor-reactive T cells to be unresponsive to immune suppressive features of the tumor, but also results in autoimmunity, and second the usage of CD19-chimeric antigen receptor (CAR)-engineered T cells, which yielded up to 90% complete remissions in end-stage leukemia patients (Maude et al., N Engl J Med 2014; 371:1507-1517). Both examples show the clinical relevance of using T cells to fight cancerous diseases. TCR-engineered T cells have advantages over aforementioned approaches. Employing TCRs targeting antigens either exclusively expressed by tumor cells (tumor specific antigens, TSA) or with a restricted expression pattern or level in healthy tissue (tumor associated antigens) leads to reduced on-target-toxicity and autoimmunity compared to the global effects of checkpoint inhibitors. In contrast to CARs, which recognize surface epitopes on tumor and/or healthy cells, TCRs recognize extracellular and intracellular processed linear epitopes presented by molecules of the major histocompatibility complex (MHC), thus broadening the target-antigen population for therapy. T cells can also recognize and destroy non-cancerous stromal cells surrounding and nutritioning the tumor cells by a mechanism called cross-presentation, in which tumor cell-derived antigens are taken up by stromal cells and presented via MHC-molecules. Several studies suggest that the simultaneous usage of CD4⁺ and CD8⁺ T cells might lead to improved therapeutic outcome by different mechanism (Caserta et al., Crit Rev Immunol, 2012; 32(2):97-126; Qin et al., Immunity, 2000; 12(6):677-686; Schietinger et al., J. Exp. Med., 2010; 207(11):2469-2477). Although CAR T cells are potent killers of tumors of hematopoietic origin, TCR gene-therapy is most promising in eradicating solid tumors, e.g. melanoma, colon and lung cancer. However, the availability of TCRs restricts the widespread use of this type of therapy so far.

The most common approach to identifying antigen-specific TCRs is based on *in vitro* stimulations of peripheral blood T cells from healthy donors. Alternatively, blood from cancer patients can be used as well as tumor-infiltrating lymphocytes, which are available in limited amounts and only in solid tumors. Deep sequencing of such samples allows the rapid identification of TCR genes included but does not allow connecting correct alpha-beta-TCR combinations. Thus, prediction of TCR specificity and antigen-recognition remains impossible and has to be tested extensively by re-expressing all possible alpha-beta-combinations. The introduction of an antigen-specific enrichment step reduces the amount of false combinations, but might also introduce a bias towards certain TCRs by overstimulation and/or activation-induced cell death. Such an enrichment step is most commonly performed by addition of peptides to the T cell culture binding to MHC-molecules and activating peptide-reactive T cells, which then expand and increase in relation to other T cell clonotypes. Also long *ex vivo* cultures might introduce a second bias towards T cells with a higher proliferative capacity (e.g. naive T cells). In silico predicted peptides might not be naturally present in tumors, thus leading to activation of peptide-specific T cells, which are not applicable for therapy.

Other *in vitro* stimulation approaches focus on natural processing and presentation of so-called true epitopes. By expressing the full-length protein in autologous antigen-presenting cells it is ensured that the T cells react to epitopes, which are naturally occurring. Nevertheless, problems associated with *ex vivo* cultures of T cells also apply here.

For isolation and molecular characterization of B cell receptors or antibodies, B cell hybridoma obtained by fusion of an immortalized cell line and a B cell have been employed (Köhler G, Milstein C. J Immunol. 2005 Mar 1;174(5):2453-5.).

The hybridoma technology was initially developed to allow the generation of large quantities of monoclonal antibodies. To do so, B cells derived from a previously immunized mouse are fused with immortalized myeloma cells (BW5147) to generate a hybridoma with a single specificity producing the desired antibody. Fusion is most commonly induced either physically by using electric pulses or chemically by using polyethylene glycol (PEG). However, historically, Sendai-virus-mediated cell-fusion emerged first. Other viruses used for induction of cell fusion are measles virus and vesicular stomatitis virus (Katoh et al., 2010, BMC biotechnology;10:37).

Using similar techniques, T cell hybridomas were also generated by fusing primary T cells with, e.g., BW5147 cells. T cell hybridomas can be obtained by fusing activated T cells with tumor cells (Kruisbeek, 2001, Curr Protoc Immunol. Chapter 3:Unit 3.14.). T cell hybridomas have been used for cloning of TCRs (Morinaga et al., 1985, PNAS 82:8163-8167;; Austin J Clin Immunol. 1(3):1015, Taniguchi and Miller, J. Exp. Med, 1978; 148:373-382; Kappler et al., J. Exp. Med, 1981; 153:1198-1214).

Each of these techniques, however, leads to unspecific hybridization resulting in homotypic fusion or unwanted heterotypic fusion events, reducing efficacy.

Successful TCR gene therapy is dependent on the availability of novel TCRs with exquisite specificity profiles. In light of this, the present inventors addressed the need to provide a new and advantageous method to identify and isolate novel receptors, in particular, antigen-specific TCRs, as well as cells useful in said method.

This problem is solved by the present invention, in particular, by the subject matter of the claims. The invention provides a method of preparing a hybridoma cell expressing a receptor, e.g., a TCR or BCR, preferably, a TCR, comprising steps of
(a) culturing an immortalized cell comprising stably integrated DNA encoding
   (i) a fusion protein comprising (1) a targeting ligand capable of specifically binding to a cell surface antigen of another cell (i.e. not expressed on said immortalized cell), and (2) a mutated paramyxovirus attachment protein, wherein said attachment protein is not capable of binding to its natural receptors;
   (ii) a paramyxovirus glycoprotein F or a derivative thereof capable of inducing fusion of the immortalized cell with another cell,
      wherein the immortalized cell expresses (i) said fusion protein and (ii) said glycoprotein F or derivative thereof,
   with the cell expressing said cell surface antigen and said receptor, wherein culture is under conditions allowing for fusion of said cells, and wherein hybridoma cells are generated by said fusion;
(b) optionally, cloning the hybridoma cells;
(c) screening the hybridoma cells for expression and/or function of the receptor, wherein fused cells expressing the receptor are selected.

The paramyxovirus family comprises seven distinct genera, of which five belong to the *paramyxovirinae* subfamily, in particular, Rubulavirus (e.g., mumps, MuV, or parainfluenza virus 5, PIV5), Avulavirus (Newcastle disease virus, NDV), Respirovirus (e.g., Sendai virus, SeV, or human parainfluenza virus 3, hPIV3), Henipavirus (e.g., Hendra virus, HeV, or Nipah virus, NiV) or Morbillivirus (e.g., measles virus, MeV, Canine distemper virus, cetacean morbillivirus, Peste-des-petits-ruminants virus, Phocine distemper vorus and Rinderpest virus). The remaining two genera are grouped in *thepneumovirinae* subfamily, which comprises viruses like respiratory syncytial virus, RSV, avian metapneumovirus, AMPV, and human metapneumovirus, HMPV.

Paramyxovirus entry occurs through virus-cell membrane fusion and is usually mediated by the attachment protein (H, HN, or G) and the fusion protein (F). All paramyxovirus attachment proteins characterized to date are homotetrameric type II integral membrane proteins. The attachment proteins of rubulaviruses, respiroviruses, and avulaviruses have both hemaglutinin (sialic acid binding) and neuraminidase (sialic acid cleaving) functions and are called HN proteins. Viruses with an HN protein use cellular surface sialic acid as their receptors with various degrees of affinity. The attachment proteins of morbilliviruses (H) lack neuraminidase activity but can bind sialic acid. However, morbilliviruses use cellular proteins, such as CD46, CD150/SLAM, and nectin-4, in the case of MeV, as receptors for attachment. Henipaviruses and members of the *pneumovirinae* subfamily have attachment proteins that do not bind sialic acid and are named G (for glycoprotein). Hendra and Nipah G bind to cellular Ephrin B2/B3 whereas previous reports have shown that pneumovirinae G proteins bind to heparan sulfate proteoglycans. In addition to mediating the initial attachment of the virus to a cell, attachment proteins of paramyxoviruses also have fusion promoting activity, as coexpression of the homotypic attachment and fusion proteins F is required for membrane fusion and viral spread to occur (Chang and Dutch, 2012, Viruses 4(4):613-636).

Throughout the invention, the employed paramyxovirus-derived glycoproteins preferably are *paramyxovirinae*-derived glycoproteins. Most preferably, Henipavirus-derived glycoproteins (e.g., Nipah virus glycoproteins) or Morbillivirus-derived glycoproteins. Measles virus-derived glycoproteins have been investigated best, and are thus most preferred. Henipavirus, in particular, Nipah virus, -derived glycoproteins have also been successfully used for retargeting. Both attachment protein and F protein are derived from one species.

Accordingly, in one embodiment, the immortalized cell comprises stably integrated DNA encoding (i) a fusion protein comprising (1) a targeting ligand capable of specifically binding to a cell surface antigen of another cell, and (2) a mutated morbillivirus, preferably, measles virus-derived attachment protein, glycoprotein H, wherein said attachment protein is not capable of binding to its natural receptors CD46, CD150/SLAM or nectin-4; and (ii) morbillivirus, preferably, measles virus glycoprotein F or a derivative thereof capable of inducing fusion of the immortalized cell with another cell.

In another embodiment, the immortalized cell comprises stably integrated DNA encoding (i) a fusion protein comprising (1) a targeting ligand capable of specifically binding to a cell surface antigen of another cell, and (2) a mutated Henipavirus, preferably, Nipah virus-derived attachment protein, glycoprotein G, wherein said attachment protein is not capable of binding to its natural receptors Ephrin B2/B3; and (ii) Henipavirus, preferably, Nipah virus glycoprotein F or a derivative thereof capable of inducing fusion of the immortalized cell with another cell.

In a third embodiment, the immortalized cell comprises stably integrated DNA encoding (i) a fusion protein comprising (1) a targeting ligand capable of specifically binding to a cell surface antigen of another cell, and (2) a mutated Respirovirus, preferably, Sendai virus-derived attachment protein, glycoprotein HN, wherein said attachment protein is not capable of binding to its natural receptors, sialic acid-containing gangliosides; and (ii) Respirovirus, preferably, Sendai virus glycoprotein F or a derivative thereof capable of inducing fusion of the immortalized cell with another cell.

It is noted that, throughout this invention, the term "fusion protein" relates to the fusion protein (i) as defined herein, comprising the targeting ligand fused to the mutated paramyxovirus attachment protein, unless it is expressly designated fusion protein F or fusion F or glycoprotein F, which relates to the paramyxovirus-derived fusion-mediating glycoprotein. In the fusion protein of the invention, the paramyxovirus-derived attachment protein may be N-termininal, and separated from the targeting ligand by a linker, e.g., a GS-linker (as shown, e.g., in SEQ ID NO: 6). The targeting ligand may be followed by a His-tag (also shown in SEQ ID NO: 6). This structure is shared by preferred fusion proteins.

The method of the invention can further comprise cloning the expressed receptor from the hybridoma cells. The method of the invention thus enables provision of novel receptors and nucleic acids encoding them, in particular TCR or BCR. One of the advantages of the method of the invention is that, by virtue of the targeting ligand capable of specifically binding to a cell surface antigen of another cell, the cells from which the receptor is cloned are very efficiently preselected, e.g., to be a CD4+ or CD8+ T cell, in case the targeting ligand is capable of specifically binding to CD4 or CD8, respectively.

An immortalized cell may be used in the method of the invention. It comprises stably integrated DNA encoding
(i) a fusion protein comprising (1) a targeting ligand capable of specifically binding to a cell surface antigen of another cell (i.e. not expressed on said immortalized cell), wherein the targeting ligand is selected from the group comprising an scFv and a DARPin (Designed Ankyrin Repeat Protein), and (2) a mutated paramyxovirus attachment protein, wherein said attachment protein is not capable of binding to its natural receptors (e.g. CD46 or CD150 or nectin-4 in the case of morbillivirus glycoprotein H, such as measles virus glycoprotein H);
(ii) paramyxovirus (e.g., morbillivirus, such as measles virus) glycoprotein F or a derivative thereof capable of inducing fusion of the immortalized cell with another cell,
and expressing (i) said fusion protein and (ii) said glycoprotein F or derivative thereof. Said paramyxovirus preferably is measles virus.

Preferably, the immortalized cell is a lymphoma or melanoma cell, e.g., a thymoma cell such as a BW cell. For example, if the other cell expressing the cell surface antigen is a T cell, preferably, the immortalized cell is a thymoma cell. In a preferred embodiment, it is a BW4 or BW8 cell as described herein. Immortalized cells of the invention can be generated, e.g., by transposon-mediated genetic modification, which leads to stable integration of transgenic DNA. For example, the transposon system employed can be Sleeping Beauty, e.g., in the form of a pT2, pT3, pT4 or pT5 transposon (as taught, e.g., in EP application No. 16160499). Accordingly, the immortalized cell may comprise the stably integrated DNA in the context of one or more, e.g., two integrated transposons, such as a Sleeping Beauty transposon as described herein.

The immortalized cell of the invention is not capable of surviving in a selection medium such as HAT medium (Hypoxynthin, Aminopterin und Thymidin), unless it is fused with a primary cell. Preferably, the immortalized cell of the invention is not a PM-1 cell and not a packaging cell such as HEK293T cell.

Preferably, the immortalized cell also does not express a T cell receptor or B cell receptor, which facilitates cloning of such receptors using the immortalized cells.

The immortalized cell suitable for carrying out the method of the invention, in particular, for preparing hybridoma cells expressing TCR or BCR or cloning such receptors, may comprise stably integrated DNA encoding
(i) a fusion protein comprising (1) a targeting ligand capable of specifically binding to CD4 or CD8 or a cell surface antigen specifically expressed by a B cell, and (2) a mutated paramyxovirus attachment protein, wherein said paramyxovirus attachment protein is not capable of binding to its natural receptors (e.g. CD46, CD150 or nectin-4 in the case of morbillivirus glycoprotein H, such as measles virus glycoprotein H));
(ii) a paramyxovirus ( e.g., morbillivirus, such as measles virus) glycoprotein F or a derivative thereof capable of inducing fusion of the immortalized cell with another cell,
wherein the immortalized cell expresses (i) said fusion protein and (ii) said glycoprotein F or derivative thereof. Preferably, the targeting ligand is capable of specifically binding to CD4 or CD8, to enable preparation of cells expressing TCR, or for cloning TCR.

Alternatively, the targeting ligand is capable of specifically binding to CD19, CD20 or CD22 to enable preparation of cells expressing BCR or for cloning BCR.

For example, immortalized cells of the invention, useful, e.g., for cloning T cell receptors, can be prepared by modifying a BW5148 TCR-/- thymoma line using Sleeping Beauty transposon systems (transposon and transposase). A construct used for genetic modification may e.g. comprise:
MPSV LIR - intron - attachment protein (e.g., morbillivirus, in particular, measles virus H protein) - anti-CD4-scFv or anti-CD8-scFv - His - MPSV RIR

The following sequences are the nucleotide sequences encoding the constructs, between the left inverted repeat (LIR) and right inverted repeat (RIR):
SEQ ID NO: 1 construct for anti-mouse CD4-scFv
SEQ ID NO: 2 construct for anti- mouse CD8-scFv
SEQ ID NO: 3 construct for anti-human CD4-DARPin
SEQ ID NO: 4 construct for anti- human CD8-scFv
MPSV LIR - intron - F protein - IRES - mCD90.2 - MPSV RIR (SEQ ID NO: 5)

MPSV stand for myeloproliferative sarcoma virus. IRES stands for internal ribosomal entry site. H means hemagglutinin and F protein stands for the fusion-mediating protein of the paramyxovirus.

Amino acid sequences of preferred encoded fusion protein and glycoprotein F are
SEQ ID NO: 6 anti-mouse CD4-scFv - mutated measles virus glycoprotein H (Hd18-variant)
SEQ ID NO: 7 anti- mouse CD8-scFv - mutated measles virus glycoprotein H(Hd18-variant)
SEQ ID NO: 8 anti-human CD4-DARPin - mutated measles virus glycoprotein H (Hd18-variant)
(SEQ ID NO: 9 anti- human CD8-scFv - mutated measles virus glycoprotein H
F protein, Fcd30-variant (SEQ ID NO: 10)

In general, both the fusion protein and the glycoprotein F, preferably, measles virus glycoprotein F, or a derivative thereof, are expressed under the control of a suitable constitutive or inducible, preferably, constitutive promotor such as MPSV. Different or identical promotors may be used.

Both the fusion protein and the glycoprotein F can be fused or expressed in linkage with a selectable marker protein, e.g., separated by an IRES or an 2A-linker (e.g. P2A, F2A, T2A, etc.), to facilitate selection of cells with stably integrated DNA of both constructs (e.g. a His-tag and mCD90.2, respectively). Cells having both constructs stably integrated can then be isolated, e.g., by magnetic cell sorting based on antibodies to the His-tag and mCD90.2 (Thyl .2, SEQ ID NO: 13). Alternative tags or methods of selection can of course also be used.

For example, selection can also take place, e.g., with antibodies to the respective measles virus glycoproteins and/or with the cell surface antigen to which the targeting ligand is directed, optionally, in soluble form. For example, FACSorting may be used for selecting immortalized cells of the invention.

Both fusion protein (comprising attachment protein and targeting ligand) and glycoprotein F can either be expressed from the same construct or from separate constructs.

In one embodiment, said targeting ligand is capable of specifically binding to CD4, wherein said targeting ligand preferably is anti-CD4-scFv or anti-CD4-DARPin. Accordingly, said immortalized cell is capable of fusion with a CD4+ cell.

In another embodiment, said targeting ligand is capable of specifically binding to CD8, wherein said targeting ligand preferably is anti-CD8-scFv or anti-CD8-DARPin. Accordingly, said immortalized cell is capable of fusion with a CD8+ cell.

In a third embodiment, said targeting ligand is capable of specifically binding to a cell surface protein specifically expressed by a B cell, such as CD 19, CD20 or CD22, preferably, an scFv targeted to any of these cell surface proteins. Accordingly, said immortalized cell is capable of fusion with a B-cell. In this context, specifically expressed by a cell type (e.g., a B cell) means that the cell surface protein is not expressed by other cell types (e.g., T cells). For example, CD38 is not specifically expressed by a B cell, as it is also expressed by T cells and Natural killer cells.

The targeting ligand may be capable of specifically binding to another cell surface antigen, which preferably is specifically expressed on a cell of interest, wherein the cell of interest typically expresses a protein which is to be analyzed and/or cloned. Such a cell of interest may also be, e.g., a gamma delta T cell.

Typically, the targeting ligand does not specifically bind to the receptor which is to be analyzed and/or cloned. However, it is also possible to target cells with a defined receptor specificity, wherein, as a targeting ligand, an antigenic protein domain or epitope bound by the receptor, in that case, typically, the BCR, is employed as part of the fusion protein. This could serve, e.g., to target cells and receptors responsible for an immune response, e.g., an autoimmune response to said protein bound by the receptor.

The targeting ligand may be capable of specifically binding to a cell surface antigen of cell which is selected from the group comprising human cells, mouse cells, rat cells, rabbit cells, guinea pig cells, hamster cells, goat cells, sheep cells, bovine cells, equine cells, avian cells, monkey cells, ape cells or camel cells. The immortalized cell may be from the same species or another species as the cell on the surface of which the cell surface antigen is expressed.

The targeting ligand may be capable of specifically binding to a cell surface antigen of a human cell, wherein the targeting ligand preferably is an scFv or a DARPin. For example, the targeting ligand may be capable of specifically binding to human CD4 (e.g., anti-human-CD4-DARPin). For example, the targeting ligand may be capable of specifically binding to human CD8 (e.g., anti-human-CD8-scFv).

In a preferred embodiment, the targeting ligand is capable of specifically binding to a cell surface antigen of a mouse cell, wherein the targeting ligand preferably is an scFv or a DARPin. For example, the targeting ligand may be capable of specifically binding to mouse CD4 (e.g., anti-mouse -CD4-scFv). For example, the targeting ligand may be capable of specifically binding to mouse CD8 (e.g., anti- mouse -CD8-scFv).

The targeting ligand may also be a DARPin, i.e., a Designed Ankyrin Repeat Protein. Generation of DARPin directed to different ligands is known in the art. Suitable DARPins for targeting to CD4+ T cells are e.g. disclosed in Zhou et al., 2015. J. Immunol. 195: 2493-2501, e.g., DARPin 29.2 for targeting to human CD4+ cells, or DARPin 57.2 for targeting to human or rhesus macaque CD4+. Sequences of exemplary suitable DARPins, which can be employed in the fusion protein of the invention instead of scFv, are found in SEQ ID NO: 3 or 8.

Alternative targeting ligands capable of mediating specific binding to a cell surface antigen can of course also be used, e.g., an alternative antibody or antibody derivative, receptor or receptor-ligand or their derivatives. Derivatives will typically consist of or comprise the domain of the protein capable of specifically binding to the cell surface antigen.

The fusion protein further comprises a mutated paramyxovirus, preferably, morbillivirus attachment protein which is not capable of binding to its natural ligand(s). Said attachment protein may be measles virus glycoprotein H, wherein said mutated measles virus glycoprotein H is not capable of binding to CD46 (complement regulatory protein) or CD150 (signaling lymphocyte activation molecule, SLAM) or nectin-4. Such mutated paramyxovirus attachment protein may be designated blinded paramyxovirus attachment protein, e.g., such measles virus glycoprotein H is also designated blinded measles virus glycoprotein H, and it may be, e.g., HmutΔ18. Suitable mutated measles virus glycoprotein H is e.g., disclosed in Zhou et al., 2012. Blood 120:4334-4342 or Zhou et al., 2015. J. Immunol. 195: 2493-2501). Zhou et al., 2012 also teaches a suitable fusion protein with anti-human CD8-scFv. Zhou et al., 2015. J. Immunol. 195: 2493-2501 teaches suitable fusion protein with DARPins targeted to human and/or rhesus macaque CD4. Both teach suitable measles virus glycoprotein F. So does Funke et al., 2008. Mol. Ther. 16:1427-1436. WO 2008/037458 A2 teaches suitable measles virus glycoprotein H and measles virus glycoprotein F and suitable derivatives thereof which may be encoded in part of the stably integrated DNA of the immortalized cell of the present invention.

The mutated paramyxovirus, preferably, morbillivirus or measles virus glycoprotein H may have the sequence of amino acid 1-597 of SEQ ID NO: 6 or at least 80%, at least 90%, at least 95% or at least 99% amino acid identity thereto. SEQ ID NO: 20-34 are comprised in preferred H-protein tail-variants Hd14, Hd15, Hd16, Hd17, Hd18, Hd19, Hd20, Hd21, Hd22, Hd23, Hd24, Hd21+1A, Hd24+4A, Hd26+6A and Hd30+10A, respectively, wherein the provided amino acids replace the wild type sequence of measles virus glycoprotein H provided in SEQ ID NO: 19. Hd18 is most preferred.

The paramyxovirus, preferably, morbillivirus or measles virus glycoprotein F preferably has the sequence of amino acid SEQ ID NO: 10, or at least 80%, at least 90%, at least 95% or at least 99% amino acid identity thereto. This corresponds to the Fcd30 variant.

SEQ ID NO: 36 and 37 are comprised in preferred variants Fd24 and Fd30 of measles virus F protein variants, respectively, wherein the provided amino acids replace the wild type sequence of measles virus glycoprotein F provided in. SEQ ID NO: 35. Said proteins are preferably expressed as transmembrane proteins, so that they can mediate contact to another cell. The immortalized cell is, by virtue of the expressed proteins, capable of fusion with a cell expressing said surface antigen, in particular, with T cell or B cell, preferably, with a CD4+ or CD8+ T cell.

Preferably, the immortalized cell of the present invention further is capable of expressing a detectable marker selected from the group comprising a fluorescent protein, beta-galactosidase and an antibiotic resistance gene, preferably, GFP, under the functional control of an inducible expression element, wherein expression of said detectable marker is inducible by ligation of a receptor expressed by a cell expressing said cell surface antigen.

The detectable marker is preferably selected from the group of fluorescent proteins, e.g., GFP, CFP, YFP, eGFP, eYFP, zoanFP, drFP583, preferably, it is GFP. A preferred GFP is shown in SEQ ID NO: 17, with a preferred nucleic acid construct in SEQ ID NO: 18.

The inducible expression element may be a promotor or enhancer or a combination thereof. Preferably, said detectable marker is inducible by NFAT (Nuclear Factor of Activated T-cells), and the inducible expression element can accordingly be induced by NFAT.

Preferably, the detectable marker can be expressed under the functional control of a heterologous expression element.

In a preferred embodiment, said receptor expressed by a cell expressing said cell surface antigen is a TCR or a BCR, preferably a TCR. Activation of the TCR or BCR leads to activation of NFAT, i.e., in that case, the detectable marker may be inducible by NFAT. Accordingly, in this context, the targeting ligand is preferably capable of specifically binding an antigen expressed by T cells, such as CD4 or CD8. The immortalized cells of this embodiment are especially useful for cloning T cell receptors, as activation of the TCR by recognition of the antigen (in context of the appropriate MHC) leads to expression of the detectable marker and simplifies screening of the hybridoma cells.

If said receptor expressed by a cell expressing said cell surface antigen is a B cell receptor (BCR), activation of the BCR e.g. leads to activation of NFAT, i.e., in that case, the detectable marker may be inducible by NFAT. Accordingly, in this context, the targeting ligand is capable of specifically binding an antigen expressed by B cells, such as CD 19, CD20 or CD22. The immortalized cells of this embodiment are useful for cloning B cell receptors, as activation of the BCR by recognition of the recognized antigen leads to expression of the detectable marker and simplifies screening of the hybridoma cells.

If the receptor expressed by a cell expressing said cell surface antigen is an orphan receptor, i.e. a receptor for which a specific ligand is not yet known, it is preferably known (or a reasonably guess is possible, e.g., based on the structure of the receptor and/or potential associated proteins or second messengers) what inducible expression elements may be activated by receptor binding, so that a detectable marker may be expressed under the functional control of said inducible expression element if the receptor is contacted by its ligand. Accordingly, such an immortalized cell, or hybridoma cells derived therefrom after fusion with a cell expressing said receptor and said cell surface antigen may be useful for identification of ligands of orphan receptors. Contact with an appropriate ligand and activation of the receptor then leads to expression of the detectable marker and provides a read-out system for the ligand.

Accordingly, after fusion with the immortalized cell of the invention capable of expressing the detectable marker as described herein, ligation of a receptor expressed by the cell expressing said cell surface antigen leads to expression of the detectable marker protein, which facilitates selection of activated cells and analysis of receptor, e.g., TCR specificity.

If the immortalized cell of the invention does not comprise such a detectable marker, selection of cells activated by receptor ligation is possible e.g., by analysis of expressed cytokines (e.g., IL-2, IL-4, and/or IFN-γ) or cell surface activation markers. Exemplary suitable methods for detection are ELISA or FACS, respectively. However, the process is significantly facilitated by the use of a detectable marker according to the invention.

Preferably, the immortalized cell of the invention further is capable of expressing an anti-apoptotic molecule selected from the group comprising Bcl-XL (mBcl-XL: SEQ ID NO: 14) and Bcl-2, preferably, Bcl-2. Alternative cellular anti-apoptotic molecules are e.g. Survivin, P35, XIAP, crmA, Mcl-1, BAG-1, c-myc, mutant p53 and others. Beyond that, also, a viral oncogene may be used as anti-apoptotic molecule, e.g. SV40 large T , EBV genes (EBNA-1, EBNA-2, LMP), HPV16 E6/7, Adenovirus E1A and others. Anti-apoptotic molecules enhance survival of cells expressing said marker, and thus provide a selective advantage.

Preferably, expression of said anti-apoptotic molecule is linked with expression of a co-receptor capable of functionally interacting with a T cell receptor expressed by a cell expressing said cell surface antigen, wherein said co-receptor is selected from the group comprising CD4 (hCD4: SEQ ID NO: 15) and CD8 (hCD8a: SEQ ID NO: 16), and wherein said co-receptor preferably is human. The inventors have shown that, after fusion of the immortalized cell of the invention with a T cell, the endogenous co-receptor CD4 or CD8 of the T cell is often lost. Accordingly, in that case, the expression of the co-receptor aids T cell signaling.

In the case of high affinity TCR, also designated co-receptor independent TCR, the co-receptor is not required. Thus, immortalized cells without the anti-apoptotic molecule linked with expression of the co-receptor can be useful for specific provision of co-receptor independent TCR.

Otherwise, in particular, for isolation of TCR from CD8+ T cells, provision of a co-receptor, expression of which is linked to expression of an anti-apoptotic molecule, significantly raises efficiency of the method of the invention. Linkage of expression can for example be achieved by expression on one mRNA, wherein the regions encoding the different proteins are separated by a 2A element (e.g. P2A, F2A, T2A, etc.). Expression of the mRNA preferably is under control of a constitutive promotor.

Preferred constructs are provided in SEQ ID NO: 11 (SB-MP71-hCD4-P2A-mBcl-xl) and SEQ ID NO: 12 (SB-MP71-hCD8a-P2A-mBcl-xl).

The invention also relates to a method of preparing a hybridoma cell expressing a receptor, e.g., a TCR or BCR, preferably, a TCR, comprising steps of
(a) culturing an immortalized cell of the invention with a cell expressing said cell surface antigen and said receptor, wherein culture is under conditions allowing for fusion of said cells, wherein hybridoma cells are generated by said fusion;
(b) optionally, cloning the hybridoma cells;
(c) screening the hybridoma cells for expression and/or function of the receptor, wherein fused cells expressing the receptor are selected.

If the receptor is a TCR restricted to MHC II, the cell expressing said antigen is a CD4+ T cell and said targeting ligand may be anti-CD4-scFv. If the receptor is a TCR restricted to MHC II and the cell expressing said antigen is a CD4+ T cell, said targeting ligand may also be another targeting ligand specifically binding CD4 such as a DARPin.

Alternatively, if the receptor is a TCR restricted to MHC I, the cell expressing said antigen a CD8+ T cell and said targeting ligand may be anti-CD8-scFv. If the receptor is a TCR restricted to MHC I and the cell expressing said antigen a CD8+ T cell, said targeting ligand may also be another targeting ligand specifically binding CD8 such as a DARPin.

The receptor can also be another receptor of interest which is to be analysed, e.g., for signaling, for expression elements induced upon ligation, or for ligand binding (e.g., for an orphan receptor).

The expressing said cell surface antigen and said receptor may be a primary cell, normally, a non-immortalized cell such as a B cell or T cell, but it may also be a tumor cell, e.g., a tumor cell isolated from a patient.

Preferably, the cell expressing said cell surface antigen and said receptor is a T cell prepared from a subject. The subject can be, e.g., a mammal or a bird, e.g., mouse, human, rat, rabbit, guinea pig, hamster, goat, sheep, cow, horse, monkey, ape or camel, preferably, mouse or human. It can also be a mammal, e.g., a mouse with a humanized TCR or BCR repertoire. Preferably, the subject, and the cell, thus comprises a human receptor.

In one embodiment, said subject has been immunized against an antigen, and the receptor is a BCR specifically directed against said antigen.

In one embodiment, said subject has been immunized against an antigen, and the receptor is a TCR specifically directed against said antigen. Said cell expressing said TCR may be a murine T cell expressing a murine TCR, a murine T cell expressing a human TCR; or a human T cell, preferably, a human T cell isolated from a tumor from said subject. Human or murine T cells can also be isolated from peripheral blood or lymph nodes. Murine T cells can easily be prepared from spleen, or a mixture of splenocytes can be employed in the method of the invention. The T cell may also be a T cell from a T cell library. It is one advantage of the method of the invention that isolation of the cells expressing the receptor and the cell surface molecule to which the targeting ligand is directed from a mixture of cells is not required, as the targeting ligand enables specific fusion and hybridoma cell generation.

Preferably, the cells expressing said TCR are activated before step a, e.g., by stimulation with antibodies against CD3 and CD28. Of course, activation can also be non-specific stimulation or activation with antigen presenting cells presenting an antigen recognized by the TCR of interest.

In another embodiment, said subject has been immunized against an antigen, and the receptor is a BCR specifically directed against said antigen. The subject can be, e.g., a mammal or a bird, e.g., a mouse or a human. It can also be a mammal, e.g., a mouse with a humanized BCR repertoire.

Of course, the skilled person would be aware of the required species specificities, e.g., of immortalized cell and targeting ligand, depending on the specificities of the system in which the method is carried out, in particular, the species of the cell expressing said cell surface antigen and said receptor and the particular surface antigen.

Culture conditions allowing for fusion of said cells are, e.g., RPMI cell culture medium supplemented with 10% fetal calf serum (FCS), 1% antibiotics/antimycotica (Penicillin, Streptomycin), 1% sodium pyruvate, 1% essential amino acids, Interleukin-2 (40U/ml) and preconditioned medium (20%). Cells are initially cultured in 100µl medium containing the above mentioned supplements. On day 1 of the coculture, 50µl of identical medium supplemented with 3xHAT is added. On day 2 of the coculture, 50µl of identical medium supplemented with 1xHT is added. Subsequently, medium is exchanged or added whenever medium is consumed, indicated by change of color of the medium. Our data indicates that the efficiency of hybridoma formation is significantly increased by addition of conditioned medium. To do so, freshly isolated splenocytes of wt-mice were activated using Concanavalin A (2 µg/ml) and murine IL-7 (1 ng/ml). 48 hours later the supernatant was harvested, filtrated, aliquoted and frozen at -80°C.

Cloning the hybridoma cells is preferably performed on a statistical basis, wherein a suitable number of cells are cultured in one well of a culture plate so that on average, one clone of hybridoma cells per well develops. It is also possible to culture the cells in bulk culture and, optionally, clone the cells after screening, however, in this case, the number of receptors and cells analyzed does not allow any conclusions on the quantitative representation of different receptors, e.g., TCR, in the sample from which the cells, e.g., T cells, were derived. Furthermore, the method is more efficient if a cloning step is included after step a, because double analysis of TCR derived from the same clone is avoided.

Selection can be direct by selection for the receptor or indirect, e.g., via effects of expression and activation of the receptor, e.g., expression of a detectable marker as described herein, cytokine production or expression of activation markers.

Preferably, in any of the methods of the invention, the immortalized cells are cells capable of expressing a detectable marker selected from the group comprising a fluorescent protein, beta-galactosidase and an antibiotic resistance gene, as described herein, under the functional control of an inducible expression element, wherein expression of said detectable marker is inducible by ligation of said receptor expressed by said cell expressing said cell surface antigen, and screening comprises selecting for expression of the detectable marker. Preferably, said detectable marker is GFP or another fluorescent protein.

Preferably, the screening comprises contacting the fused cells with antigen-presenting cells (APC), especially if the receptor is a TCR. Said APC may be, e.g., tumor cells or APC such as professional APC (e.g., B cells, dendritic cells or macrophages) presenting, in the context of MHC I (in particular for CD8 restricted TCR) and/or MHC II (in particular for CD4 restricted TCR), a peptide from an antigen, to which the TCR is directed. The APC may be, e.g., a professional APC modified to express or loaded with an antigen of interest which may be a peptide or a protein suitable for processing by the APC.

If analysis and optionally cloning of a human TCR is intended, the APC may be, e.g., a human APC or a murine APC expressing human MHC I and/or MHCII. The APC may be derived from a sample of a subject, which may be a tumor sample or healthy tissue, or it may be a tumor cell line. Optionally, screening is sequentially carried out with a plurality of different tumor samples and/or tumor cell lines and/or antigens.

If the receptor is a BCR, the screening preferably comprises contacting the fused cells with antigen. Antigen-binding cells may then be directly selected or screened for expression of a detectable marker as defined herein or other activation dependent markers, e.g., cytokines or cell-surface activation markers.

Optionally, the method allows for screening for high or low affinity receptors, preferably, high affinity receptors, by affinity-based screening, wherein, preferably the screening is carried out based on a detectable marker as described herein. The affinity-based screening may be carried out by titrating the amount of ligands recognizable by the receptor, e.g., the amount of complexes of MHC and peptide for a TCR. High affinity receptors can be detected at a low amount of ligands. For example, the amount of peptide presented on antigen-presenting cells can be titrated.

The invention also relates to a method of preparing a nucleic acid encoding a receptor, e.g., a TCR or BCR, preferably, a TCR, comprising the method of the invention of preparing a hybridoma cell expressing said receptor, the method further comprising d) isolating the nucleic acid encoding the receptor from the selected cells.

The method can also comprise amplifying and or cloning said nucleic acid into a vector, e.g., a sequencing and/or expression vector.

The invention also relates to a method for preparing a receptor, e.g., a TCR, comprising carrying out the method of preparing a nucleic acid encoding a receptor of the invention and expressing said receptor, wherein the method optionally comprises isolating the receptor.

Said method for preparing a receptor may also be a method for preparing a B cell receptor or an antigen-binding derivative thereof. In the context of the invention, this encompasses preparation of an antigen-binding fragment of a B cell receptor, which e.g., does not necessarily express the transmembrane regions of the BCR or the constant regions of the antibody. For example, an IgG antibody, a Fab fragment, a Fab₂ fragment, an scFv or similar derivatives of a BCR can be prepared, e.g, after suitable genetic modification of the nucleic acid encoding the BCR or after enzymatic digestion of the BCR and purification of the desired fragment. Optionally, fusion of BCR variable regions to T cell activating molecule domains can be carried out (e.g. CD3zeta, CD28, 4-1BB aka CD137), leading to preparation of a chimeric antigen receptor (CAR) construct.

The invention also relates to a method for preparing a receptor-positive cell, in particular, a TCR-or CAR-positive cell, comprising preparing a nucleic acid encoding a receptor identified by a method of the invention, wherein the isolated nucleic acid encodes a TCR, a BCR, and modifying a T cell with the nucleic acid encoding the isolated receptor. Modification can be stable or transient, e.g., transfection, viral gene transfer or electroporation with a transposon system such as Sleeping Beauty.

Said method optionally comprises formulating said TCR or CAR-positive cell in a composition suitable for administration to a subject. For example, such methods can be of therapeutic benefit in case the subject has a tumor, and the CAR or TCR is directed to a free antigen or a pMHC-complex from the tumor. Of course, such methods can also be of therapeutic benefit in case the subject has an infectious disease, and the CAR or TCR is directed to free antigen or a pMHC-complex from the agent causing the infectious disease. Preferably, for treatment of a human subject, the transfected T cell is a human T cell, in particular, a human T cell derived from said subject. For treatment of a murine subject, the transfected T cell is a murine T cell, in particular, a murine T cell derived from said mouse or a syngeneic mouse.

The invention also provides a receptor-positive cell, in particular, a TCR- or CAR- positive cell obtainable by the method of the invention for preparing a receptor-positive cell, as well as a pharmaceutical composition comprising the same, e.g., for treatment of a tumor or an infectious disease.

The invention also relates to a method for quantitatively and/or qualitatively analyzing the TCR or BCR receptor repertoire of cells in a sample, comprising analyzing the nucleic acids encoding the receptors of a plurality of hybridoma cells obtained by carrying out the method of the invention comprising step b), wherein said receptors preferably are TCR. This can be useful, e.g., for analyzing the immune status of a specific patient at different time points, or for comparing the immune status of two or more patients at one or more time points.

The invention also relates to a hybridoma cell expressing a receptor (e.g., a BCR or TCR, preferably a TCR), wherein the cell is capable of expressing a detectable marker as defined herein, preferably, GFP, under the functional control of an inducible expression element, wherein expression of said detectable marker is inducible by ligation of the receptor,
wherein the hybridoma cell comprises at least one of the following:
(i) stably integrated DNA encoding a fusion protein comprising (1) a targeting ligand capable of specifically binding to a cell surface antigen of another cell, wherein the targeting ligand is selected from the group comprising an scFv and a DARPin, and (2) a mutated paramyxovirus attachment protein (preferably, measles virus glycoprotein H or Nipah virus glycoprotein G), wherein said mutated attachment protein is not capable of binding to its natural receptors (e.g. CD46 and CD150 for measles virus); wherein the hybridoma cell optionally expresses said fusion protein; and/or
(ii) stably integrated DNA encoding paramyxovirus (preferably, measles virus or Nipah virus) glycoprotein F or a derivative thereof capable of inducing fusion of the lymphoma cell with another cell; wherein the hybridoma cell optionally expresses said glycoprotein F or derivative thereof,
(iii) and, in case the receptor is a TCR, a co-receptor capable of functionally interacting with the TCR selected from the group consisting of CD4 and CD8, and an anti-apoptotic molecule.

Said hybridoma cell optionally is a cell obtainable from the method of the invention of preparing a hybridoma cell expressing a receptor. The invention is also directed to a hybridoma cell obtainable from the method of the invention of preparing a hybridoma cell expressing a receptor.

While hybridomas are genetically instable, the DNA of (i) and (ii) above is stably integrated and should not be lost, even if expression may not be detectable anymore. The inventors could show that, often, hybridoma cells obtained by the method of the invention loose expression of either attachment protein (in the form of the fusion protein described above) or F protein. However, typically, expression of one of said proteins, often, F protein, is maintained and can be detected. Preferably, the hybridoma cell expresses at least the fusion protein (a) and the co-receptor of (c), or the hybridoma cell expresses at least the glycoprotein F of (b) and the co-receptor of (c). Of course, the hybridoma cell may also express at least the fusion protein (a), the glycoprotein F of (b) and the co-receptor of (c). The hybridoma cell of the invention additionally preferably comprises the detectable marker as defined herein.

The invention is also directed to use of said hybridoma cells as indicator cells, e.g., for analyzing tumor material, e.g., tumor material from a patient, to determine presence of an antigen detectable by the receptor.

Key advantages of the present invention are:
- Pre-selection for cell subsets, in particular, T cell subsets (e.g., CD4 or CD8).
- Screening for antigen-specificity before isolation of TCRs is possible.
- Depending on the priming-approach, targeting true epitopes is possible.
- The method leads to increased hybridization efficiency without homotypic fusion.
- The method allows for generation of T cell libraries for analysis of immune reactions.
- The method is easy-to-perform, with a low-tech approach.

In the following, the invention is exemplified by figures and examples, which are intended to illustrate, not to limit the invention. All references cited herein are herewith fully incorporated.

### Figure legends

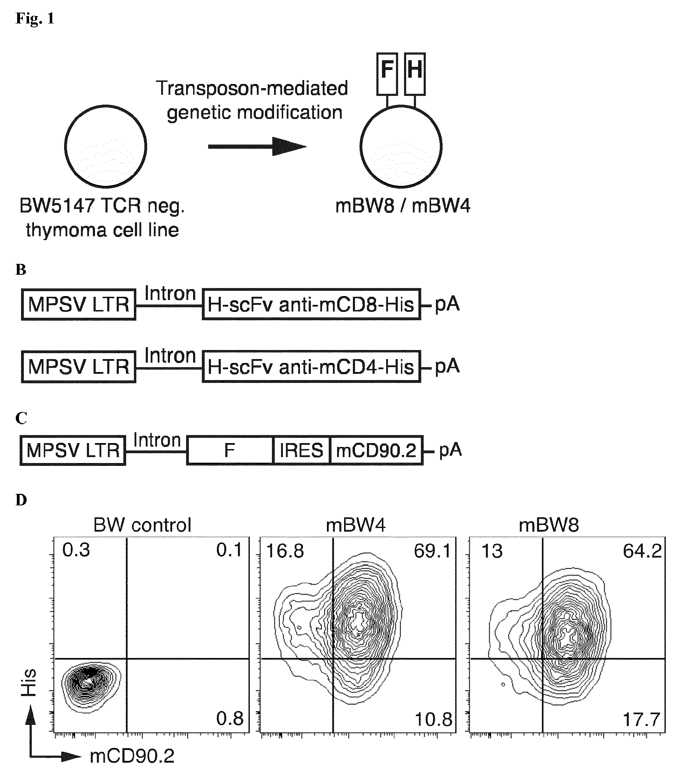

**Fig. 1** Generation of cell lines stably expressing redirected measles virus fusogenic proteins for the production of T cell hybridomas. **A** The BW5147 TCR-/- thymoma cell line was genetically modified using the transposon-based Sleeping Beauty vector system to generate the cell lines mBW8 and mBW4. **B, C** Transgene cassettes of the transposon vectors used to generate mBW8 or mBW4 cells. **D** Analysis of surface expression of histidine-tagged H protein and mCD90.2 on mBW4, mBW8 and unmodified BW cells (BW control) by flow cytometry. Myeloproliferative sarcoma virus long terminal repeat (MPSV LTR), mutated glycoprotein H (H), single-chain variable fragment (scFv), polyhistidine tag (His), polyadenylation signal (pA), glycoprotein protein F (F), internal ribosome entry (IRES), mouse CD90.2 surface receptor (mCD90.2).

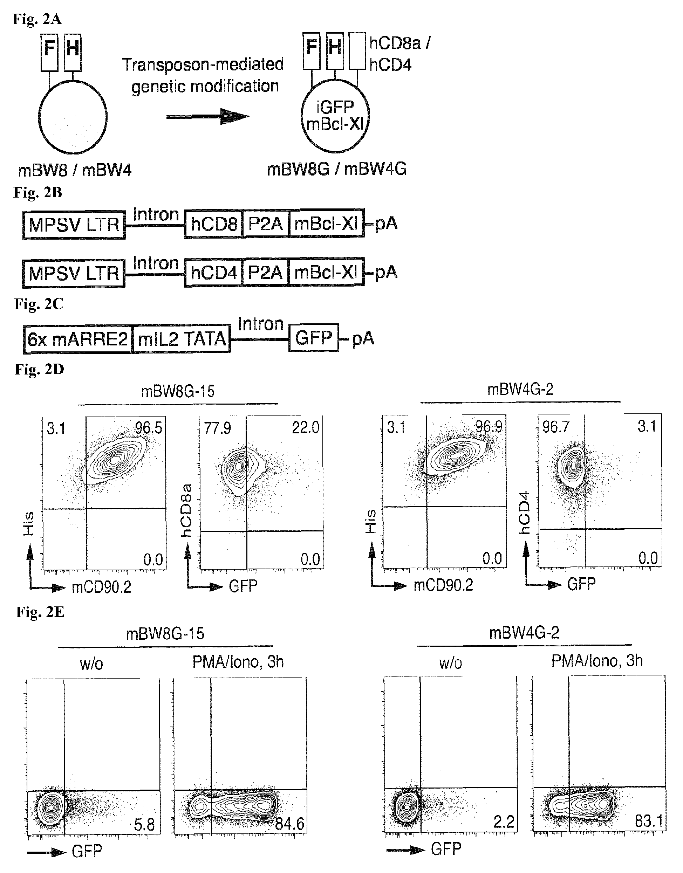

**Fig. 2** Additional genetic modifications were added to the mBW8 and mBW4 cell lines to facilitate the analysis of TCR specificity using T cell hybridomas. **A** The new cell lines mBW8G and mBW4G were generated using the mBW8 and mBW4 lines, respectively. **B, C** Transgene cassettes of the transposon vectors used to generate the mBW8G or mBW4G cell lines. **D** Selected clones of the mBW8G and mBW4G cell lines were analyzed by flow cytometry for GFP and surface expression of histidine-tagged H protein, mCD90.2, human CD8 or CD4. **E** The clonal cell lines mBW8G-15 and mBW4G-2 were stimulated for 3 h with PMA/Iono and induced GFP expression was analyzed by flow cytometry. Myeloproliferative sarcoma virus long terminal repeat (MPSV LTR), human CD8a (hCD8a), human CD4 (hCD4), inducible GFP (iGFP), mouse Bcl-Xl (mBcl-Xl), porcine techovirus 2A peptide linker (P2A), polyadenylation signal (pA), mouse antigen receptor recognition element 2 (mARRE2), mouse interleukin 2 TATA box (mIL2 TATA), green fluorescent protein (GFP), ionomycin (Iono), Phorbol 12-myristate 13-acetate (PMA), mouse CD90.2 surface receptor (mCD90.2).

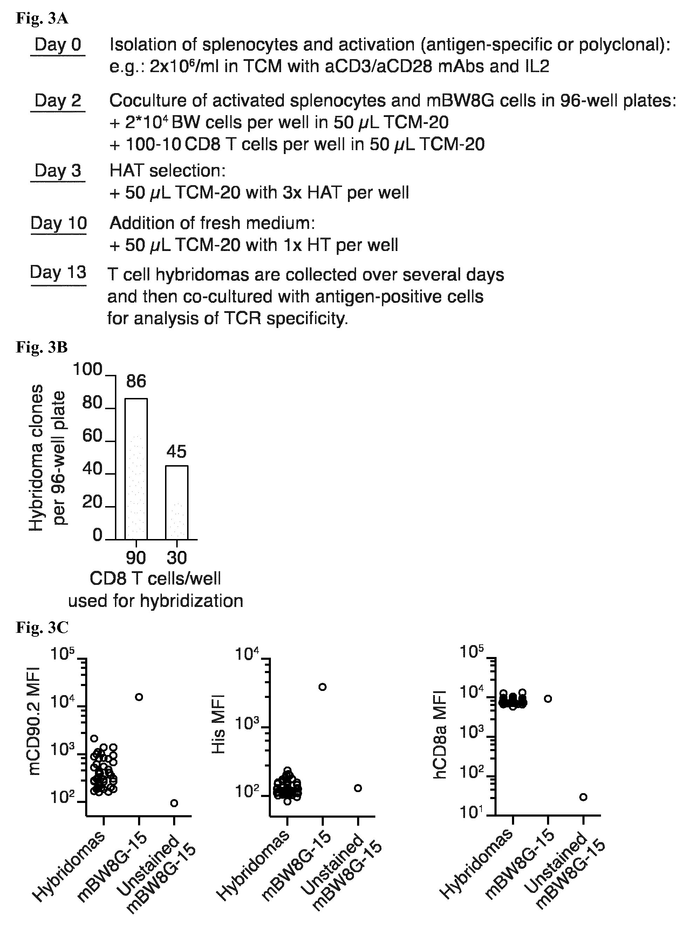

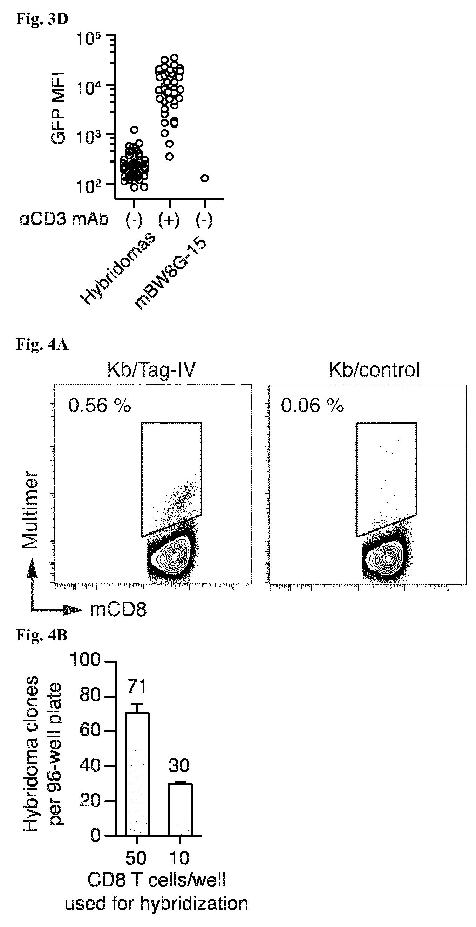

**Fig. 3** Generation and characterization of mouse T cell hybridomas. **A** Outline of a method of the invention for generation of mouse T cell hybridomas using mBW8G-15 cells. **B** Shown is the number of T cell hybridomas per 96-well plate generated by using 90 or 30 CD8 T cells for hybridization with 2x10⁴ mBW8G-15 cells per well. Representative data of one plate per condition is shown. **C** Analysis of T cell hybridomas derived from B6.SJL mice (mCD90.1) by flow cytometry. The stained and unstained parental mBW8G-15 cell line is shown for comparison. **D** The T cell hybridoma cultures where split in two and one half was stimulated overnight with plate-bound mCD3-specific mAb. The next day GFP expression of stimulated and unstimulated hybridomas was analyzed by flow cytometry. T cell medium supplemented with 20% conditioned medium (TCM-20), green fluorescent protein (GFP), HAT (hypoxanthine, aminopterin and thymidine), HT (hypoxanthine and thymidine).

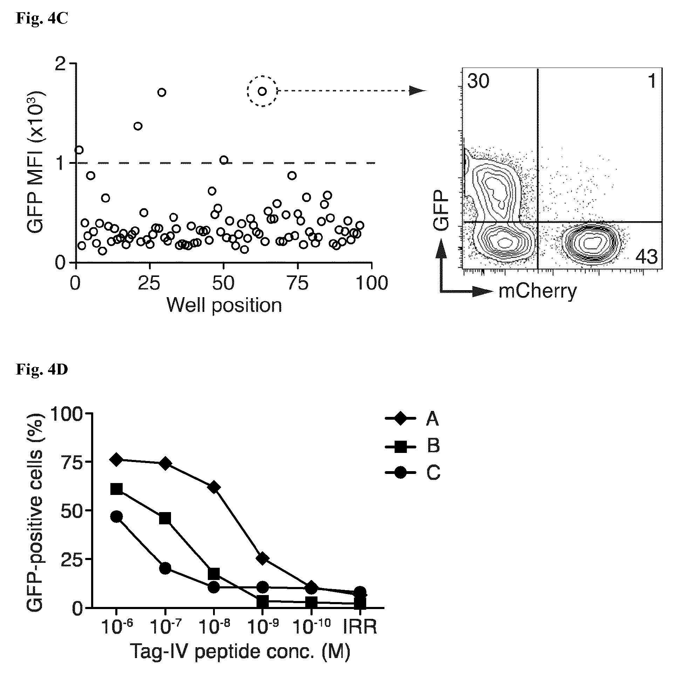

**Fig. 4** Generation of a CD8 T cell hybridoma library from immunized mice and identification of antigen-specific hybridomas. **A** B6 mice were immunized with irradiated tumor cells expressing the simian virus 40 (SV40) large T antigen (Tag). 10 days later the splenocytes were isolated and stained for SV40 Tag epitope IV-specific T cells. **B** Shown is the number of T cell hybridomas per 96-well plate generated by using 50 or 10 CD8 T cells for hybridization with 2x10⁴ mBW8G-15 cells per well. Data of two plates per condition are shown (± SD). **C** The T cell hybridoma library was interrogated using Kb- and mCherry-expressing T2 cells loaded with peptide IV (10⁻⁶ M). After overnight co-culture in 96-well plates the cells were analyzed by flow cytometry. A GFP MFI of the mCherry-negative cells of over 1000 (marked by the dashed line) was counted as a positive reaction. **D** Selected T cell hybridomas A, B and C were analyzed using Kb- and mCherry-expressing T2 cells loaded with titrated amounts of peptide IV (10⁻⁶ - 10⁻¹⁰ M).

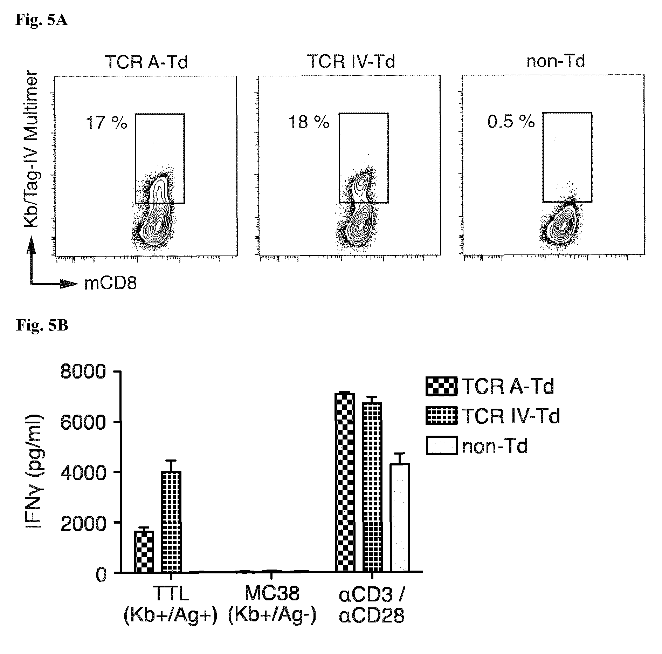

**Fig. 5** Transgenic expression of TCR genes isolated from T cell hybridoma A in transduced mouse T cells confers SV40 Tag epitope IV-specific reactivity. A Mouse T cells (B6) were transduced with γ-retroviral MP71 vectors encoding either the αβ TCR that was isolated from hybridoma A (TCR A) or a SV40 Tag epitope IV-specific control TCR (TCR IV). The transduced T cells were stained with a specific Kb/Tag IV multimer and analyzed by flow cytometry. **B** The cytokine secretion of the transduced T cells after overnight incubation with antigen-positive TTL and -negative MC38 tumor cells was measured by ELISA.

### Examples

Cell lines stably expressing the mutated glycoprotein H and glycoprotein F were generated using the transposon-based Sleeping Beauty vector system (Fig. 1A). TCR-negative BW5147 cells were first transfected by electroporation with two plasmids encoding the transposon and transposase and then 10 days later magnetically sorted for the genetically modified cells. In a first step transposon vectors encoding either the His-tagged and mutated glycoprotein H targeting mouse CD8 or CD4 (Fig. 1B) were used. Afterwards the two resulting cell lines were magnetically sorted using an αHis-specific mAb. In a second step, the glycoprotein F was introduced into the two cell lines using another transposon vector in which the F protein was linked via an internal ribosome entry (IRES) site to mouse CD90.2. The latter was used afterwards as a marker to enrich the genetically modified cells. The resulting two cell lines were named mBW8 and mBW4. The glycoprotein F was expressed by over 70% of the cells in both cell lines as indicated by staining for the marker mCD90.2 (Fig. 1D). At the same time, over 70% of the mBW8 and mBW4 cells expressed the mutated glycoprotein H targeting mouse CD8 or CD4, respectively, as demonstrated by staining for the His-tag (Fig. 1D).

To facilitate the analysis of TCR specificity, the mBW8 and mBW4 cell lines were further modified by the introduction of the human CD8 or CD4 co-receptor, respectively (Fig. 2A). Both receptor genes were coupled via a 2A linker to the anti-apoptotic mouse Bcl-Xl protein (Fig. 2B). Another transposon vector was co-transfected into both the cell lines encoding a NFAT-regulated GFP marker gene (Fig. 2C). The resulting cell lines mBW8G and mBW4G were sorted by FACS for the expression of His-tagged H protein, mCD90.2 and human CD8 or CD4. Next, single-cell clones were established by limited dilution from the sorted bulk cultures. The clones mBW8G-15 and mBW4G-2 (Fig. 2D) were selected for further investigation based on the surface expression of the transgenic proteins. GFP expression could be induced in both clonal cell lines after a short incubation with PMA/Ionomycin (Fig. 2E).

T cell hybridoma libraries were generated by co-culture of activated mouse T cells with either the mBW8G-15 or mBW4G-2 cell line in 96-well plates (Fig. 3A). Mouse T cell medium (TCM) consisting of RPMI 1640 GlutaMAX medium supplemented with 10 % heat-inactivated FCS, 100 IU/ml Penicillin/Streptomycin, 1 mM sodium pyruvate and 1x non-essential amino acids was used to culture the mouse activated splenocytes. The modified BW cell lines were resuspended in fresh T cell medium supplemented with 20% conditioned medium (TCM-20) and transferred into the 96-well plates. Conditioned medium was generated by activating wt-splenocytes using Concanavalin A (2 µg/ml) and murine IL-7 (1 ng/ml). 48 hours later the supernatant was harvested, filtrated, aliquoted and stored at -80°C until use as additive in TCM leading to TCM-20. Next, the percentage of CD8 T cells within the population of activated T cells was determined by flow cytometry, a defined amount of CD8 T cells was resuspended in TCM-20 and transferred into the 96-well plates to the modified BW cells. The following day, TCM-20 supplemented with HAT and one week later, TCM-20 supplemented with HT was added. About two weeks after the start of the protocol, the T cell hybridomas were collected on several consecutive days by transfer into new 96-well plates with fresh TCM-20. Using this protocol and the mBW8-15 cell line, a hybridization efficiency of 1-3 % was achieved (Fig. 3B and 4B). The T cell hybridomas expressed almost no His-tagged H protein and reduced amounts of mutated F protein as indicated by staining for mCD90.2 on the cell surface (Fig. 3C). In contrast, the human CD8 co-receptor was expressed at high levels that were comparable to the expression level on the parental mBW8-15 cell line (Fig. 3C). This data indicates that stable expression of this transgene in the T cell hybridomas was achieved by coupling the human CD8 gene to the anti-apoptotic factor Bcl-Xl. Furthermore, the GFP expression was induced upon stimulation of the T cell hybridomas using a CD3-specific mAb, demonstrating that almost all T cell hybridomas express TCR on their surface and that the library can be interrogated by providing TCR stimulation and analyzing GFP expression (Fig. 3D).

As a proof-of-principle, a CD8 T cell hybridoma library was generated from an immunized mouse using the mBW8G-15 cell line, and an antigen-specific TCR was isolated. A C57BL/6 mouse was immunized with irradiated tumor cells expressing the simian virus 40 (SV40) large T antigen (Tag). 10 days later, the splenocytes were isolated and 0.56 % SV40 Tag epitope IV-specific T cells were detected in the CD8 compartment by staining with the cognate MHC multimer (Fig. 4A). The splenocytes were activated with CD3/CD28-specific mAbs for two days and then fused with mBW8G-15 cells in 96-well plates following the protocol described above (Fig. 3A). A hybridization efficiency of 1.5 - 3 % was achieved (Fig. 4B). The T cell hybridomas were collected on new 96-well plates and then split into two plates. On half of the plates Kb- and mCherry-expressing T2 cells loaded with 10⁻⁶ M peptide IV were added. On the other half of the plates, fresh TCM-20 was added to expand the T cell hybridomas. GFP expression was analyzed in the co-culture cells by flow cytometry after overnight incubation (Fig. 4C). Some of the hybridomas that showed GFP expression after the co-culture were further investigated using Kb-and mCherry-expressing T2 cells loaded with titrated amounts of peptide IV (Fig. 4D). Subsequently, the αβ TCR chains from hybridoma clone A that showed the highest GFP induction were isolated by 5'RACE (rapid amplification of cDNA ends) method.

To investigate the antigen specificity of the isolated TCR A, this TCR and a SV40 Tag epitope IV-specific TCR (TCR IV) were re-expressed in primary mouse T cells. Antigen-specific staining of the TCR-transduced T cells was demonstrated by using the cognate Kb/Tag IV multimer (Fig. 5A). Moreover, TCR A-transduced T cells secreted IFNγ upon co-culture with antigen-positive but not antigen-negative tumor cells (Fig. 5B). In conclusion, this data demonstrated that the T cell hybridoma library generated with the mBW8G-15 cell line from the immunized mouse was successfully used to isolate a TCR that recognizes the tumor cell line in an antigen-specific fashion.

## Claims

1. A method of preparing a hybridoma cell expressing a receptor, comprising steps of
(a) culturing an immortalized cell comprising stably integrated DNA encoding
(i) a fusion protein comprising (1) a targeting ligand capable of specifically binding to a cell surface antigen of another cell, and (2) a mutated paramyxovirus attachment protein, wherein said attachment protein is not capable of binding to its natural receptors;
(ii) a paramyxovirus glycoprotein F or a derivative thereof capable of inducing fusion of the immortalized cell with another cell,
wherein the immortalized cell expresses (i) said fusion protein and (ii) said glycoprotein F or derivative thereof,
with a cell expressing said cell surface antigen and said receptor, wherein culture is under conditions allowing for fusion of said cells, and wherein hybridoma cells are generated by said fusion;
(b) optionally, cloning the hybridoma cells;
(c) screening the hybridoma cells for expression and/or function of the receptor, wherein at least one hybridoma cell expressing the receptor is selected.

2. A method of preparing a nucleic acid encoding a receptor, comprising preparing a hybridoma cell expressing a receptor by the method of claim 1 and further comprising d) isolating the nucleic acid encoding the receptor from the selected cell(s).

3. The method of any of claims 1 or 2, wherein the receptor is a TCR, wherein the cell expressing said cell surface antigen is a CD4+ T cell and said targeting ligand is selected from the group comprising anti-CD4-scFv and anti-CD4 DARPin.

4. The method of any of claims 1 or 2, wherein the receptor is a TCR, the cell expressing said cell surface antigen is a CD8+ T cell and said targeting ligand is selected from the group comprising anti-CD8-scFv and anti-CD8 DARPin.

5. The method of any of claims 3 or 4, wherein the screening comprises contacting the hybridoma cells with antigen-presenting cells.

6. The method of any of claims 1 or 2, wherein the receptor is a BCR, the targeting ligand is capable of specifically binding to a cell surface antigen of a B cell, and the cell expressing said cell surface antigen is a B cell,
wherein the screening preferably comprises contacting the hybridoma cells with antigen.

7. The method of any of claims 1 to 6, wherein the immortalized cell further is capable of expressing a detectable marker selected from the group comprising a fluorescent protein, beta-galactosidase and an antibiotic resistance gene, preferably, GFP, under the functional control of an inducible expression element, wherein expression of said detectable marker is inducible by ligation of a receptor expressed by a cell expressing said cell surface antigen,
and wherein screening comprises selecting for expression of the detectable marker, wherein said detectable marker preferably is inducible by NFAT.

8. The method of any of claims 1-7, wherein the immortalized cell further is capable of expressing an anti-apoptotic molecule selected from the group comprising Bcl-XL, Bcl-2, Survivin, P35, XIAP, crmA, Mcl-1, BAG-1, c-myc, mutant p53, SV40 large T, EBV EBNA-1, EBV EBNA-2, EBV LMP, HPV16 E6, HPV16 7 and Adenovirus E1A.
wherein, if the receptor is a TCR, preferably, expression of said anti-apoptotic molecule is linked with expression of a co-receptor selected from the group comprising CD4 and CD8, which is capable of functionally interacting with the TCR expressed by the T cell expressing said cell surface antigen.

9. The method of any of claims 1-8, which allows for screening for high or low affinity receptors, preferably, high affinity receptors, by affinity-based screening,
wherein, preferably, the affinity-based screening is carried out by titrating the amount of ligands recognizable by the receptor.

10. The method of any of claims 1-9, wherein the paramyxovirus is a morbillivirus, and the attachment protein is a mutated morbillivirus glycoprotein H, wherein said glycoprotein H is not capable of binding to its natural receptors; wherein the morbillivirus preferably is measles virus, and the natural receptors of glycoprotein H are CD46, CD150/SLAM and nectin-4.

11. A method for preparing a receptor, comprising carrying out the method of preparing a nucleic acid encoding a receptor of any of claims 2-10 and expressing said receptor, wherein the method optionally comprises isolating the receptor.

12. A method for preparing a TCR-positive cell, comprising preparing a nucleic acid encoding a receptor by a method of any of claims 2-5 or 7-10, wherein the isolated nucleic acid encodes a TCR, and transfecting a T cell with a nucleic acid encoding the isolated receptor,
wherein the method optionally comprises formulating said TCR-positive cell in a composition suitable for administration to a subject.

13. A method for quantitatively and/or qualitatively analyzing the TCR or BCR receptor repertoire of cells in a sample, comprising analyzing the nucleic acids encoding the receptors of a plurality of hybridoma cells obtained by carrying out the method of any of claims 1-10 comprising step b), wherein said receptors preferably are TCR.

14. An immortalized cell suitable for carrying out the method of any of claims 1-13, the immortalized cell comprising stably integrated DNA encoding
(i) a fusion protein comprising (1) a targeting ligand capable of specifically binding to CD4 or CD8 or a cell surface antigen specifically expressed by a B cell, and (2) a mutated paramyxovirus attachment protein, wherein said morbillivirus glycoprotein H is not capable of binding to its natural receptors;
(ii) a paramyxovirus glycoprotein F or a derivative thereof capable of inducing fusion of the immortalized cell with another cell,
and expressing (i) said fusion protein and (ii) said glycoprotein F or derivative thereof.

15. The immortalized cell of claim 14, wherein the immortalized cell further is capable of expressing a detectable marker selected from the group comprising a fluorescent protein, beta-galactosidase and an antibiotic resistance gene, preferably, GFP under the functional control of an inducible expression element, wherein expression of said detectable marker is inducible by ligation of a TCR, wherein, optionally, said detectable marker is inducible by NFAT.

16. The immortalized cell of any of claims 14 or 15, wherein the immortalized cell further is capable of expressing an anti-apoptotic molecule selected from the group comprising Bcl-XL and Bcl-2,
wherein, preferably, expression of said anti-apoptotic molecule is linked with expression of a co-receptor capable of functionally interacting with the TCR receptor expressed by a T cell expressing said CD4 or CD8, wherein said co-receptor is CD4 or CD8.

17. A hybridoma cell expressing a receptor, wherein the cell is capable of expressing a detectable marker selected from the group comprising a fluorescent protein, preferably, GFP, under the functional control of an inducible expression element, wherein expression of said detectable marker is inducible by ligation of the receptor,
wherein the hybridoma cell comprises at least one of the following:
(a) stably integrated DNA encoding a fusion protein comprising (1) a targeting ligand capable of specifically binding to a cell surface antigen of another cell, and (2) a mutated paramyxovirus attachment protein, wherein said mutated attachment protein is not capable of binding to its natural receptors; wherein the hybridoma cell optionally expresses said fusion protein; and/or
(b) stably integrated DNA encoding paramyxovirus glycoprotein F or a derivative thereof capable of inducing fusion of the lymphoma cell with another cell; wherein the hybridoma cell optionally expresses said glycoprotein F or derivative thereof,
(c) in case the receptor is a TCR, a co-receptor capable of functionally interacting with the TCR selected from the group consisting of CD4 and CD8, and an anti-apoptotic molecule.
wherein said hybridoma cell optionally is a cell obtainable from the method of any of claims 1 or 3-10,
or a hybridoma cell obtainable from the method of any of claims 1 or 3-10.


SEQUENCE LISTING

<110> Max-Delbrueck-Centrum fuer Molekulare Medizin
      Humboldt-UniversitÃ¤t zu Berlin

<120> Method for the generation of T cell hybridoma for cloning and analysis
of receptors
using engineered measles virus fusogenic glycoproteins


<130> MDC16861EP

<160> 37

<170> BiSSAP 1.3

<210> 1
<211> 3964
<212> DNA
<213> Artificial Sequence

<220>
<223> construct for anti-mouse CD4-scFv

<400> 1
cagttgaagt cggaagttta catacactta agttggagtc attaaaactc gtttttcaac      60

tactccacaa atttcttgtt aacaaacaat agttttggca agtcagttag gacatctact     120

ttgtgcatga cacaagtcat ttttccaaca attgtttaca gacagattat ttcacttata     180

attcactgta tcacaattcc agtgggtcag aagtttacat acactaagtt gactgtgcct     240

ttaaacagct tggaaaattc cagaaaatga tgtcatggct ttagaagctt gagaagttca     300

gatcaaggtt aggaacagag agacagcaga atatgggcca aacaggatat ctgtggtaag     360

cagttcctgc cccggctcag ggccaagaac agttggaaca gcagaatatg ggccaaacag     420

gatatctgtg gtaagcagtt cctgccccgg ctcagggcca agaacagatg gtccccagat     480

gcggtcccgc cctcagcagt ttctagagaa ccatcagatg tttccagggt gccccaagga     540

cctgaaatga ccctgtgcct tatttgaact aaccaatcag ttcgcttctc gcttctgttc     600

gcgcgcttct gctccccgag ctcaataaaa gagcccacaa cccctcactc ggcgcgccag     660

tcctccgata gactgcgtcg cccgggtacc cgtgttctca ataaaccctc ttgcagttgc     720

atccgactcg tggtctcgct gttccttggg agggtctcct ctgagtgatt gactgcccac     780

ctcgggggtc tttcatttgt ccggagaggc actgggcagg taagtatcaa ggttacaaga     840

caggtttaag gagaccaata gaaactgggc ttgtcgagac agaacgcgta attaatgcat     900

gaagactctt gcgtttctga taggcaccta ttggtcttac tgacatccac tttgcctttc     960

tctccacagg tgtccactcc cagcggccgc caccatggga agtaggatag tcattaacag    1020

agaacatctt atgattgata gaccttatgt tttgctggct gttctgtttg tcatgtttct    1080

gagcttgatc gggttgctag ccattgcagg aattcgactt catcgggcag ccatctacac    1140

cgcagagatc cataaaagcc tcagcaccaa tctagatgta actaactcaa tcgagcatca 1200
ggtcaaggac gtgctgacac cactcttcaa aatcatcggt gatgaagtgg gcctgaggac 1260
acctcagaga ttcactgacc tagtgaaatt catctctgac aagattaaat tccttaatcc 1320
ggatagggag tacgacttca gagatctcac ttggtgtatc aacccgccag agagaatcaa 1380
attggattat gatcaatact gtgcagatgt ggctgctgaa gagctcatga atgcattggt 1440
gaactcaact ctactggaga ccagaacaac caatcagttc ctagctgtct caaagggaaa 1500
ctgctcaggg cccactacaa tcagaggtca attctcaaac atgtcgctgt ccctgttaga 1560
cttgtattta ggtcgaggtt acaatgtgtc atctatagtc actatgacat cccagggaat 1620
gtatggggga acttacctag tggaaaagcc taatctgagc agcaaaaggt cagagttgtc 1680
acaactgagc atgtaccgag tgtttgaagt aggtgttatc agaaatccgg gtttgggggc 1740
tccggtgttc catatgacaa actatcttga gcaaccagtc agtaatgatc tcagcaactg 1800
tatggtggct ttgggggagc tcaaactcgc agccctttgt cacggggaag attctatcac 1860
aattccctat cagggatcag ggaaaggtgt cagcttccag ctcgtcaagc taggtgtctg 1920
gaaatcccca accgacatgc aatcctgggt ccccttatca acggatgatc cagtgataga 1980
caggctttac ctctcatctc acagaggtgt tatcgctgac aaccaagcaa aatgggctgt 2040
cccgacaaca cgaacagatg acaagttgcg aatggagaca tgcttccaac aggcgtgtaa 2100
gggtaaaatc caagcactct gcgagaatcc cgagtgggca ccattgaagg ataacaggat 2160
tccttcatac ggggtcttgt ctgttgatct gagtctgaca gttgagctta aaatcaaaat 2220
tgcttcggga ttcgggccat tgatcacaca cggttcaggg atggacctat acaaatccaa 2280
ccacaacaat gtgtattggc tgactatccc gccaatgaag aacctagcct taggtgtaat 2340
caacacattg gagtggatac cgagattcaa ggttagtccc gcactcttca ctgtcccaat 2400
taaggaagca ggcggagact gccatgcccc aacataccta cctgcggagg tggatggtga 2460
tgtcaaactc agttccaatc tggtgattct acctggtcaa gatctccaat atgttttggc 2520
aacctacgat acttccgcgg ttgaacatgc tgtggtttat tacgtttaca gcccaagccg 2580
cctatcgtct tacttttatc cttttaggtt gcctataaag ggggtcccca tcgaattaca 2640
agtggaatgc ttcacatggg accaaaaact ctggtgccgt cacttctgtg tgcttgcgga 2700
ctcagaatct ggtggacata tcactcactc tgggatggtg ggcatgggag tcagctgcac 2760
agtcacccgg gaagatggaa ccaatggtgg aggtggctct ggtggaggag gctctggagg 2820
tggtggatca gcggcccagc cggccgaagt gcagctggtg cagtctggcg ccgagctcgt 2880
gaaacctggc gcctctgtga agctgagctg caaggtgtcc gactacaaca tccggcggac 2940
ctacatgcac tgggtgcgcc agaggcctgg caagggcctg gaatggatcg gcagaatcga 3000
ccccgccaac ggcaacacca tctacggcga gaagttcaag agcaaggcca ccctgaccgc 3060

cgacaccagc agcaacaccg cctatatgca gctgagccag ctgaagtccg acgacaccgc 3120

catctactac tgcgccatcg gcgtgcagta cctggactat tggggccagg gcaccaccgt 3180

gacagtgtct agcggaggcg gaggatctgg cggaggggga tccgggggag gcggatctga 3240

tatcgtgctg acacagagcc cagccctggc cgtgtctcct ggcgagagag tgaccatcag 3300

ctgcagagcc accgagagcg tgtccaccct gatccactgg tatcagcagc ggcctggcca 3360

gcagcccaag ctgctgatct acctgaccag ccacctggac agcggcgtgc cagccagatt 3420

ttctggctct ggcagcggca ccgactttac cctgaccatc gaccctgtgg aagccgatga 3480

tacagccacc tactattgcc agcagacctg gaacgacccc tggacctttg gcggaggcac 3540

caagctggaa ctgaagagag cggccgcaag aggttctcat caccatcacc atcactaaca 3600

attcaataaa atatctttat tttcattaca tctgtgtgtt ggttttttgt gtgtaattcg 3660

gatctagctt gtggaaggct actcgaaatg tttgacccaa gttaaacaat ttaaaggcaa 3720

tgctaccaaa tactaattga gtgtatgtaa acttctgacc cactgggaat gtgatgaaag 3780

aaataaaagc tgaaatgaat cattctctct actattattc tgatatttca cattcttaaa 3840

ataaagtggt gatcctaact gacctaagac agggaatttt tactaggatt aaatgtcagg 3900

aattgtgaaa aagtgagttt aaatgtattt ggctaaggtg tatgtaaact tccgacttca 3960

actg 3964

<210> 2
<211> 3970
<212> DNA
<213> Artificial Sequence

<220>
<223> construct for anti- mouse CD8-scFv

<400> 2
cagttgaagt cggaagttta catacactta agttggagtc attaaaactc gtttttcaac 60

tactccacaa atttcttgtt aacaaacaat agttttggca agtcagttag gacatctact 120

ttgtgcatga cacaagtcat ttttccaaca attgtttaca gacagattat ttcacttata 180

attcactgta tcacaattcc agtgggtcag aagtttacat acactaagtt gactgtgcct 240

ttaaacagct tggaaaattc cagaaaatga tgtcatggct ttagaagctt gagaagttca 300

gatcaaggtt aggaacagag agacagcaga atatgggcca aacaggatat ctgtggtaag 360

cagttcctgc cccggctcag ggccaagaac agttggaaca gcagaatatg ggccaaacag 420

gatatctgtg gtaagcagtt cctgccccgg ctcagggcca agaacagatg gtccccagat 480

gcggtcccgc cctcagcagt ttctagagaa ccatcagatg tttccagggt gccccaagga 540

cctgaaatga ccctgtgcct tatttgaact aaccaatcag ttcgcttctc gcttctgttc 600

gcgcgcttct gctccccgag ctcaataaaa gagcccacaa cccctcactc ggcgcgccag 660
tcctccgata gactgcgtcg cccgggtacc cgtgttctca ataaaccctc ttgcagttgc 720
atccgactcg tggtctcgct gttccttggg agggtctcct ctgagtgatt gactgcccac 780
ctcgggggtc tttcatttgt ccggagaggc actgggcagg taagtatcaa ggttacaaga 840
caggtttaag gagaccaata gaaactgggc ttgtcgagac agaacgcgta attaatgcat 900
gaagactctt gcgtttctga taggcaccta ttggtcttac tgacatccac tttgcctttc 960
tctccacagg tgtccactcc cagcggccgc caccatggga agtaggatag tcattaacag 1020
agaacatctt atgattgata gaccttatgt tttgctggct gttctgtttg tcatgtttct 1080
gagcttgatc gggttgctag ccattgcagg aattcgactt catcgggcag ccatctacac 1140
cgcagagatc cataaaagcc tcagcaccaa tctagatgta actaactcaa tcgagcatca 1200
ggtcaaggac gtgctgacac cactcttcaa aatcatcggt gatgaagtgg gcctgaggac 1260
acctcagaga ttcactgacc tagtgaaatt catctctgac aagattaaat tccttaatcc 1320
ggatagggag tacgacttca gagatctcac ttggtgtatc aacccgccag agagaatcaa 1380
attggattat gatcaatact gtgcagatgt ggctgctgaa gagctcatga atgcattggt 1440
gaactcaact ctactggaga ccagaacaac caatcagttc ctagctgtct caaagggaaa 1500
ctgctcaggg cccactacaa tcagaggtca attctcaaac atgtcgctgt ccctgttaga 1560
cttgtattta ggtcgaggtt acaatgtgtc atctatagtc actatgacat cccagggaat 1620
gtatggggga acttacctag tggaaaagcc taatctgagc agcaaaaggt cagagttgtc 1680
acaactgagc atgtaccgag tgtttgaagt aggtgttatc agaaatccgg gtttgggggc 1740
tccggtgttc catatgacaa actatcttga gcaaccagtc agtaatgatc tcagcaactg 1800
tatggtggct ttgggggagc tcaaactcgc agccctttgt cacggggaag attctatcac 1860
aattccctat cagggatcag ggaaaggtgt cagcttccag ctcgtcaagc taggtgtctg 1920
gaaatcccca accgacatgc aatcctgggt ccccttatca acggatgatc cagtgataga 1980
caggctttac ctctcatctc acagaggtgt tatcgctgac aaccaagcaa aatgggctgt 2040
cccgacaaca cgaacagatg acaagttgcg aatggagaca tgcttccaac aggcgtgtaa 2100
gggtaaaatc caagcactct gcgagaatcc cgagtgggca ccattgaagg ataacaggat 2160
tccttcatac ggggtcttgt ctgttgatct gagtctgaca gttgagctta aaatcaaaat 2220
tgcttcggga ttcgggccat tgatcacaca cggttcaggg atggacctat acaaatccaa 2280
ccacaacaat gtgtattggc tgactatccc gccaatgaag aacctagcct taggtgtaat 2340
caacacattg gagtggatac cgagattcaa ggttagtccc gcactcttca ctgtcccaat 2400
taaggaagca ggcggagact gccatgcccc aacataccta cctgcggagg tggatggtga 2460
tgtcaaactc agttccaatc tggtgattct acctggtcaa gatctccaat atgttttggc 2520

aacctacgat acttccgcgg ttgaacatgc tgtggtttat tacgtttaca gcccaagccg 2580

cctatcgtct tacttttatc cttttaggtt gcctataaag ggggtcccca tcgaattaca 2640

agtggaatgc ttcacatggg accaaaaact ctggtgccgt cacttctgtg tgcttgcgga 2700

ctcagaatct ggtggacata tcactcactc tgggatggtg ggcatgggag tcagctgcac 2760

agtcacccgg gaagatggaa ccaatggtgg aggtggctct ggtggaggag gctctggagg 2820

tggtggatca gcggcccagc cggccgaggt gcagctggtg gaatctgggg gaggcttagt 2880

gcagcctgga aggtccctga aactctcctg tgcagcctca ggattcactt tcagtaacta 2940

ttacatggcc tgggtccgcc aggctccaac gaagggtctg gagtgggtcg catacattaa 3000

tactggtggt ggtaccactt actatcgaga ctccgtgaag ggccgattca ctatctccag 3060

agatgatgca aaaagcaccc tatacctgca aatggacagt ctgaggtctg aggacacggc 3120

cacttattac tgtacaacag ccatagggta ctactttgat tactggggcc aaggagtcat 3180

ggtcacagtc tcctcaggcg gaggcggctc gggcggaggc ggatccggcg gcggaggcag 3240

cgacatccag atgacacagt ctccagcttc cctgtctgca tctctgggag aaactgtctc 3300

catcgaatgt ctagcaagtg aggacatata cagttattta gcatggtatc agcagaagcc 3360

agggaaatct cctcaggtcc tgatctatgc tgcaaatagg ttgcaagatg gggtcccatc 3420

acggttcagt ggcagtggat ctggcacaca gtattctctc aagatcagcg gcatgcaacc 3480

tgaagatgaa ggggattatt tctgtctaca gggttccaag tttccgtaca cgtttggagc 3540

tgggaccaag ctggaattga aacgggcggc cgcaagaggt tctcatcacc atcaccatca 3600

ctaacaattc aataaaatat ctttattttc attacatctg tgtgttggtt ttttgtgtgt 3660

aattcggatc tagcttgtgg aaggctactc gaaatgtttg acccaagtta aacaatttaa 3720

aggcaatgct accaaatact aattgagtgt atgtaaactt ctgacccact gggaatgtga 3780

tgaaagaaat aaaagctgaa atgaatcatt ctctctacta ttattctgat atttcacatt 3840

cttaaaataa agtggtgatc ctaactgacc taagacaggg aatttttact aggattaaat 3900

gtcaggaatt gtgaaaaagt gagtttaaat gtatttggct aaggtgtatg taaacttccg 3960

acttcaactg 3970

<210> 3
<211> 3577
<212> DNA
<213> Artificial Sequence

<220>
<223> construct for anti-human CD4-DARPin

<400> 3
cagttgaagt cggaagttta catacactta agttggagtc attaaaactc gtttttcaac 60

tactccacaa atttcttgtt aacaaacaat agttttggca agtcagttag gacatctact 120

ttgtgcatga cacaagtcat ttttccaaca attgtttaca gacagattat ttcacttata 180

attcactgta tcacaattcc agtgggtcag aagtttacat acactaagtt gactgtgcct 240

ttaaacagct tggaaaattc cagaaaatga tgtcatggct ttagaagctt gagaagttca 300

gatcaaggtt aggaacagag agacagcaga atatgggcca aacaggatat ctgtggtaag 360

cagttcctgc cccggctcag ggccaagaac agttggaaca gcagaatatg ggccaaacag 420

gatatctgtg gtaagcagtt cctgccccgg ctcagggcca agaacagatg gtccccagat 480

gcggtcccgc cctcagcagt ttctagagaa ccatcagatg tttccagggt gccccaagga 540

cctgaaatga ccctgtgcct tatttgaact aaccaatcag ttcgcttctc gcttctgttc 600

gcgcgcttct gctccccgag ctcaataaaa gagcccacaa cccctcactc ggcgcgccag 660

tcctccgata gactgcgtcg cccgggtacc cgtgttctca ataaaccctc ttgcagttgc 720

atccgactcg tggtctcgct gttccttggg agggtctcct ctgagtgatt gactgcccac 780

ctcgggggtc tttcatttgt ccggagaggc actgggcagg taagtatcaa ggttacaaga 840

caggtttaag gagaccaata gaaactgggc ttgtcgagac agaacgcgta attaatgcat 900

gaagactctt gcgtttctga taggcaccta ttggtcttac tgacatccac tttgcctttc 960

tctccacagg tgtccactcc cagcggccgc caccatggga agtaggatag tcattaacag 1020

agaacatctt atgattgata gaccttatgt tttgctggct gttctgtttg tcatgtttct 1080

gagcttgatc gggttgctag ccattgcagg aattcgactt catcgggcag ccatctacac 1140

cgcagagatc cataaaagcc tcagcaccaa tctagatgta actaactcaa tcgagcatca 1200

ggtcaaggac gtgctgacac cactcttcaa aatcatcggt gatgaagtgg gcctgaggac 1260

acctcagaga ttcactgacc tagtgaaatt catctctgac aagattaaat tccttaatcc 1320

ggatagggag tacgacttca gagatctcac ttggtgtatc aacccgccag agagaatcaa 1380

attggattat gatcaatact gtgcagatgt ggctgctgaa gagctcatga atgcattggt 1440

gaactcaact ctactggaga ccagaacaac caatcagttc ctagctgtct caaagggaaa 1500

ctgctcaggg cccactacaa tcagaggtca attctcaaac atgtcgctgt ccctgttaga 1560

cttgtattta ggtcgaggtt acaatgtgtc atctatagtc actatgacat cccagggaat 1620

gtatggggga acttacctag tggaaaagcc taatctgagc agcaaaaggt cagagttgtc 1680

acaactgagc atgtaccgag tgtttgaagt aggtgttatc agaaatccgg gtttgggggc 1740

tccggtgttc catatgacaa actatcttga gcaaccagtc agtaatgatc tcagcaactg 1800

tatggtggct ttgggggagc tcaaactcgc agccctttgt cacggggaag attctatcac 1860

aattccctat cagggatcag ggaaaggtgt cagcttccag ctcgtcaagc taggtgtctg 1920

gaaatcccca accgacatgc aatcctgggt ccccttatca acggatgatc cagtgataga 1980

caggctttac ctctcatctc acagaggtgt tatcgctgac aaccaagcaa aatgggctgt 2040

cccgacaaca cgaacagatg acaagttgcg aatggagaca tgcttccaac aggcgtgtaa 2100

gggtaaaatc caagcactct gcgagaatcc cgagtgggca ccattgaagg ataacaggat 2160

tccttcatac ggggtcttgt ctgttgatct gagtctgaca gttgagctta aaatcaaaat 2220

tgcttcggga ttcgggccat tgatcacaca cggttcaggg atggacctat acaaatccaa 2280

ccacaacaat gtgtattggc tgactatccc gccaatgaag aacctagcct taggtgtaat 2340

caacacattg gagtggatac cgagattcaa ggttagtccc gcactcttca ctgtcccaat 2400

taaggaagca ggcggagact gccatgcccc aacataccta cctgcggagg tggatggtga 2460

tgtcaaactc agttccaatc tggtgattct acctggtcaa gatctccaat atgttttggc 2520

aacctacgat acttccgcgg ttgaacatgc tgtggtttat tacgtttaca gcccaagccg 2580

cctatcgtct tacttttatc cttttaggtt gcctataaag ggggtcccca tcgaattaca 2640

agtggaatgc ttcacatggg accaaaaact ctggtgccgt cacttctgtg tgcttgcgga 2700

ctcagaatct ggtggacata tcactcactc tgggatggtg ggcatgggag tcagctgcac 2760

agtcacccgg gaagatggaa ccaatgcggc ccagccggcc gacctgggta agaaactgct 2820

ggaagctgct cgtgctggtc aggacgacga agttcgtatc ctgatggcta acggtgctga 2880

cgttaacgct actgacactc ttggtcgtac tccgctacac ctggctgctc agaatggtca 2940

cctggaaatc gttgaagttc tgctgaagca cagtgctgac gttaacgcta ttgaagaggt 3000

tggtatgact ccgctgcacc tggctgttgt tgctggtcac ctggaaatcg ttgaagttct 3060

gctgaagaac ggtgctgacg ttaacgctca ggacaaattc ggtaagaccg ctttcgacat 3120

ctccatcgac tacggtaacg aggacctggc tgaaatcctg caaaagctta atgcggccgc 3180

aagaggttct catcaccatc accatcacta acaattcaat aaaatatctt tattttcatt 3240

acatctgtgt gttggttttt tgtgtgtaat tcggatctag cttgtggaag gctactcgaa 3300

atgtttgacc caagttaaac aatttaaagg caatgctacc aaatactaat tgagtgtatg 3360

taaacttctg acccactggg aatgtgatga aagaaataaa agctgaaatg aatcattctc 3420

tctactatta ttctgatatt tcacattctt aaaataaagt ggtgatccta actgacctaa 3480

gacagggaat ttttactagg attaaatgtc aggaattgtg aaaaagtgag tttaaatgta 3540

tttggctaag gtgtatgtaa acttccgact tcaactg 3577

<210> 4
<211> 3973
<212> DNA
<213> Artificial Sequence

<220>
<223> construct for anti- human CD8-scFv

<400> 4
cagttgaagt cggaagttta catacactta agttggagtc attaaaactc gtttttcaac 60

tactccacaa atttcttgtt aacaaacaat agttttggca agtcagttag gacatctact 120

ttgtgcatga cacaagtcat ttttccaaca attgtttaca gacagattat ttcacttata 180

attcactgta tcacaattcc agtgggtcag aagtttacat acactaagtt gactgtgcct 240

ttaaacagct tggaaaattc cagaaaatga tgtcatggct ttagaagctt gagaagttca 300

gatcaaggtt aggaacagag agacagcaga atatgggcca aacaggatat ctgtggtaag 360

cagttcctgc cccggctcag ggccaagaac agttggaaca gcagaatatg ggccaaacag 420

gatatctgtg gtaagcagtt cctgccccgg ctcagggcca agaacagatg gtccccagat 480

gcggtcccgc cctcagcagt ttctagagaa ccatcagatg tttccagggt gccccaagga 540

cctgaaatga ccctgtgcct tatttgaact aaccaatcag ttcgcttctc gcttctgttc 600

gcgcgcttct gctccccgag ctcaataaaa gagcccacaa cccctcactc ggcgcgccag 660

tcctccgata gactgcgtcg cccgggtacc cgtgttctca ataaaccctc ttgcagttgc 720

atccgactcg tggtctcgct gttccttggg agggtctcct ctgagtgatt gactgcccac 780

ctcgggggtc tttcatttgt ccggagaggc actgggcagg taagtatcaa ggttacaaga 840

caggtttaag gagaccaata gaaactgggc ttgtcgagac agaacgcgta attaatgcat 900

gaagactctt gcgtttctga taggcaccta ttggtcttac tgacatccac tttgcctttc 960

tctccacagg tgtccactcc cagcggccgc caccatggga agtaggatag tcattaacag 1020

agaacatctt atgattgata gaccttatgt tttgctggct gttctgtttg tcatgtttct 1080

gagcttgatc gggttgctag ccattgcagg aattcgactt catcgggcag ccatctacac 1140

cgcagagatc cataaaagcc tcagcaccaa tctagatgta actaactcaa tcgagcatca 1200

ggtcaaggac gtgctgacac cactcttcaa aatcatcggt gatgaagtgg gcctgaggac 1260

acctcagaga ttcactgacc tagtgaaatt catctctgac aagattaaat tccttaatcc 1320

ggatagggag tacgacttca gagatctcac ttggtgtatc aacccgccag agagaatcaa 1380

attggattat gatcaatact gtgcagatgt ggctgctgaa gagctcatga atgcattggt 1440

gaactcaact ctactggaga ccagaacaac caatcagttc ctagctgtct caaagggaaa 1500

ctgctcaggg cccactacaa tcagaggtca attctcaaac atgtcgctgt ccctgttaga 1560

cttgtattta ggtcgaggtt acaatgtgtc atctatagtc actatgacat cccagggaat 1620

gtatggggga acttacctag tggaaaagcc taatctgagc agcaaaaggt cagagttgtc 1680

acaactgagc atgtaccgag tgtttgaagt aggtgttatc agaaatccgg gtttgggggc 1740

tccggtgttc catatgacaa actatcttga gcaaccagtc agtaatgatc tcagcaactg 1800

tatggtggct ttgggggagc tcaaactcgc agccctttgt cacggggaag attctatcac 1860

aattccctat cagggatcag ggaaaggtgt cagcttccag ctcgtcaagc taggtgtctg 1920

gaaatcccca accgacatgc aatcctgggt ccccttatca acggatgatc cagtgataga 1980

caggctttac ctctcatctc acagaggtgt tatcgctgac aaccaagcaa aatgggctgt 2040

cccgacaaca cgaacagatg acaagttgcg aatggagaca tgcttccaac aggcgtgtaa 2100

gggtaaaatc caagcactct gcgagaatcc cgagtgggca ccattgaagg ataacaggat 2160

tccttcatac ggggtcttgt ctgttgatct gagtctgaca gttgagctta aaatcaaaat 2220

tgcttcggga ttcgggccat tgatcacaca cggttcaggg atggacctat acaaatccaa 2280

ccacaacaat gtgtattggc tgactatccc gccaatgaag aacctagcct taggtgtaat 2340

caacacattg gagtggatac cgagattcaa ggttagtccc gcactcttca ctgtcccaat 2400

taaggaagca ggcggagact gccatgcccc aacataccta cctgcggagg tggatggtga 2460

tgtcaaactc agttccaatc tggtgattct acctggtcaa gatctccaat atgttttggc 2520

aacctacgat acttccgcgg ttgaacatgc tgtggtttat tacgtttaca gcccaagccg 2580

cctatcgtct tacttttatc cttttaggtt gcctataaag ggggtcccca tcgaattaca 2640

agtggaatgc ttcacatggg accaaaaact ctggtgccgt cacttctgtg tgcttgcgga 2700

ctcagaatct ggtggacata tcactcactc tgggatggtg ggcatgggag tcagctgcac 2760

agtcacccgg gaagatggaa ccaatggtgg aggtggctct ggtggaggag gctctggagg 2820

tggtggatca gcggcccagc cggcccaggt gcagctggtg cagagcggag ccgaggataa 2880

gaaacctggc gctagcgtga aggtgtcctg caaggccagc ggcttcaaca tcaaggacac 2940

ctacatccac tgggtgcgcc aggctccagg acagggcctg gaatggatgg gccggatcga 3000

ccccgccaac gacaacaccc tgtacgccag caagttccag ggcagagtga ccatcaccgc 3060

cgacaccagc agcaacaccg cctacatgga actgagcagc ctgagaagcg aggacaccgc 3120

cgtgtactac tgtggcagag gctacggcta ctacgtgttc gaccactggg gccagggcac 3180

caccgtgaca gtgtctagcg gcggaggcgg aagtggaggc ggaggaagcg gaggcggcgg 3240

atccgacatc gtgatgaccc agagccctag cagcctgtct gccagcgtgg gcgaccgcgt 3300

gaccattacc tgccggacca gccggtccat cagccagtac ctggcctggt atcaggaaaa 3360

gcccggcaag gcccccaagc tgctgatcta cagcggcagc accctgcaga gcggcgtgcc 3420

aagcagattc agcggcagcg gctccggcac cgacttcacc ctgaccatca gcagcctgca 3480

gcccgaggac ttcgccacct actactgcca gcagcacaac gagaaccccc tgaccttcgg 3540

gcagggaaca aaggtggaga tcaagagagc ggccgcaaga ggttctcatc accatcacca 3600

tcactaacaa ttcaataaaa tatctttatt ttcattacat ctgtgtgttg gttttttgtg 3660

tgtaattcgg atctagcttg tggaaggcta ctcgaaatgt ttgacccaag ttaaacaatt 3720

taaaggcaat gctaccaaat actaattgag tgtatgtaaa cttctgaccc actgggaatg 3780

tgatgaaaga aataaaagct gaaatgaatc attctctcta ctattattct gatatttcac 3840

attcttaaaa taaagtggtg atcctaactg acctaagaca gggaattttt actaggatta 3900

aatgtcagga attgtgaaaa agtgagttta aatgtatttg gctaaggtgt atgtaaactt 3960

ccgacttcaa ctg 3973

<210> 5
<211> 4076
<212> DNA
<213> Artificial Sequence

<220>
<223> construct encoding F protein derived from MeV

<400> 5
cagttgaagt cggaagttta catacactta agttggagtc attaaaactc gtttttcaac 60

tactccacaa atttcttgtt aacaaacaat agttttggca agtcagttag gacatctact 120

ttgtgcatga cacaagtcat ttttccaaca attgtttaca gacagattat ttcacttata 180

attcactgta tcacaattcc agtgggtcag aagtttacat acactaagtt gactgtgcct 240

ttaaacagct tggaaaattc cagaaaatga tgtcatggct ttagaagctt gagaagttca 300

gatcaaggtt aggaacagag agacagcaga atatgggcca aacaggatat ctgtggtaag 360

cagttcctgc cccggctcag ggccaagaac agttggaaca gcagaatatg ggccaaacag 420

gatatctgtg gtaagcagtt cctgccccgg ctcagggcca agaacagatg gtccccagat 480

gcggtcccgc cctcagcagt ttctagagaa ccatcagatg tttccagggt gccccaagga 540

cctgaaatga ccctgtgcct tatttgaact aaccaatcag ttcgcttctc gcttctgttc 600

gcgcgcttct gctccccgag ctcaataaaa gagcccacaa cccctcactc ggcgcgccag 660

tcctccgata gactgcgtcg cccgggtacc cgtgttctca ataaaccctc ttgcagttgc 720

atccgactcg tggtctcgct gttccttggg agggtctcct ctgagtgatt gactgcccac 780

ctcgggggtc tttcatttgt ccggagaggc actgggcagg taagtatcaa ggttacaaga 840

caggtttaag gagaccaata gaaactgggc ttgtcgagac agaacgcgta attaatgcat 900

gaagactctt gcgtttctga taggcaccta ttggtcttac tgacatccac tttgcctttc 960

tctccacagg tgtccactcc cagcggccgc caccatgtcc atcatgggtc tcaaggtgaa 1020

cgtctctgcc atattcatgg cagtactgtt aactctccaa acacccaccg gtcaaatcca 1080

ttggggcaat ctctctaaga taggggtggt aggaatagga agtgcaagct acaaagttat 1140

gactcgttcc agccatcaat cattagtcat aaaattaatg cccaatataa ctctcctcaa 1200

taactgcacg agggtagaga ttgcagaata caggagacta ctgagaactg ttttggaacc 1260

aattagagat gcacttaatg caatgaccca gaatataaga ccggttcaga gtgtagcttc 1320

aagtaggaga cacaagagat ttgcgggagt agtcctggca ggtgcggccc taggcgttgc 1380

cacagctgct cagataacag ccggcattgc acttcaccag tccatgctga actctcaagc 1440

catcgacaat ctgagagcga gcctggaaac tactaatcag gcaattgagg caatcagaca 1500

agcagggcag gagatgatat tggctgttca gggtgtccaa gactacatca ataatgagct 1560

gataccgtct atgaaccaac tatcttgtga tttaatcggc cagaagctcg ggctcaaatt 1620

gctcagatac tatacagaaa tcctgtcatt atttggcccc agcttacggg accccatatc 1680

tgcggagata tctatccagg ctttgagcta tgcgcttgga ggagacatca ataaggtgtt 1740

agaaaagctc ggatacagtg gaggtgattt actgggcatc ttagagagca gaggaataaa 1800

ggcccggata actcacgtcg acacagagtc ctacttcatt gtcctcagta tagcctatcc 1860

gacgctgtcc gagattaagg gggtgattgt ccaccggcta gagggggtct cgtacaacat 1920

aggctctcaa gagtggtata ccactgtgcc caagtatgtc gcaacccaag ggtaccttat 1980

ctcgaatttt gatgagtcat cgtgtacttt catgccagag ggaactgtgt gcagccaaaa 2040

tgccttgtac ccgatgagtc ctctgctcca agaatgcctc cgggggtcca ctaagtcctg 2100

tgctcgtaca ctcgtatccg ggtcttttgg gaaccggttc attttatcac aagggaacct 2160

aatagccaat tgtgcatcaa tcctttgcaa gtgttacaca acaggaacga tcattaatca 2220

agaccctgac aagatcctaa catacattgc tgccgatcac tgcccggtag tcgaggtgaa 2280

cggcgtgacc atccaagtcg ggagcaggag gtatccagac gctgtgtact tgcacagaat 2340

tgacctcggt cctcccatat cattggagag gttggacgta gggacaaatc tggggaatgc 2400

aattgctaag ttggaggatg ccaaggaatt gttggagtca tcggaccaga tattgaggag 2460

tatgaaaggt ttatcgagca ctagcatagt ctacatcctg attgcagtgt gtcttggagg 2520

gttgataggg atccccgctt taatatgttg ctgcaggggg cgttgagaat tccgctctag 2580

aactagtgga tctccacgtg gcggctagta ctccggtatt gcggtaccct tgtacgcctg 2640

ttttatactc ccttcccgta acttagacgc acaaaaccaa gttcaataga agggggtaca 2700

aaccagtacc accacgaaca agcacttctg tttccccggt gatgtcgtat agactgcttg 2760

cgtggttgaa agcgacggat ccgttatccg cttatgtact tcgagaagcc cagtaccacc 2820

tcggaatctt cgatgcgttg cgctcagcac tcaaccccag agtgtagctt aggctgatga 2880

gtctggacat ccctcaccgg tgacggtggt ccaggctgcg ttggcggcct acctatggct 2940

aacgccatgg gacgctagtt gtgaacaagg tgtgaagagc ctattgagct acataagaat 3000

cctccggccc ctgaatgcgg ctaatcccaa cctcggagca ggtggtcaca aaccagtgat 3060

tggcctgtcg taacgcgcaa gtccgtggcg gaaccgacta ctttgggtgt ccgtgtttcc 3120

ttttatttta ttgtggctgc ttatggtgac aatcacagat tgttatcata aagcgaattg 3180

gataggatca agcttatcga taccgtcgag gccgcgccac catgaaccca gccatcagcg 3240

tcgctctcct gctctcagtc ttgcaggtgt cccgagggca gaaggtgacc agcctgacag 3300

cctgcctggt gaaccaaaac cttcgcctgg actgccgcca tgagaataac accaaggata 3360

actccatcca gcatgagttc agcctgaccc gagagaagag gaagcacgtg ctctcaggca 3420

ccctcgggat acccgagcac acgtaccgct cccgcgtcac cctctccaac cagccctata 3480

tcaaggtcct taccctagcc aacttcacca ccaaggatga gggcgactac ttttgtgagc 3540

ttcaagtctc gggcgcgaat cccatgagct ccaataaaag tatcagtgtg tatagagaca 3600

aactggtcaa gtgtggcggc ataagcctgc tggttcagaa cacatcctgg atgctgctgc 3660

tgctgctttc cctctccctc ctccaagccc tggacttcat ttctctgtga caattcaata 3720

aaatatcttt attttcatta catctgtgtg ttggtttttt gtgtgtaatt cggatctagc 3780

ttgtggaagg ctactcgaaa tgtttgaccc aagttaaaca atttaaaggc aatgctacca 3840

aatactaatt gagtgtatgt aaacttctga cccactggga atgtgatgaa agaaataaaa 3900

gctgaaatga atcattctct ctactattat tctgatattt cacattctta aaataaagtg 3960

gtgatcctaa ctgacctaag acagggaatt tttactagga ttaaatgtca ggaattgtga 4020

aaaagtgagt ttaaatgtat ttggctaagg tgtatgtaaa cttccgactt caactg 4076

<210> 6
<211> 867
<212> PRT
<213> Artificial Sequence

<220>
<221> DOMAIN
<222> 1..597
<223> H protein

<220>
<223> construct for anti-mouse CD4-scFv

<220>
<221> DOMAIN
<222> 598..612
<223> GS linker

<220>
<221> DOMAIN
<222> 613..861
<223> anti-mouse CD4-scFv

<220>
<221> DOMAIN
<222> 862..867
<223> His tag

<400> 6
Met Gly Ser Arg Ile Val Ile Asn Arg Glu His Leu Met Ile Asp Arg
1               5                   10                  15
Pro Tyr Val Leu Leu Ala Val Leu Phe Val Met Phe Leu Ser Leu Ile

                20                  25                  30
Gly Leu Leu Ala Ile Ala Gly Ile Arg Leu His Arg Ala Ala Ile Tyr
        35                  40                  45
Thr Ala Glu Ile His Lys Ser Leu Ser Thr Asn Leu Asp Val Thr Asn
    50                  55                  60
Ser Ile Glu His Gln Val Lys Asp Val Leu Thr Pro Leu Phe Lys Ile
65                  70                  75                  80
Ile Gly Asp Glu Val Gly Leu Arg Thr Pro Gln Arg Phe Thr Asp Leu
                85                  90                  95
Val Lys Phe Ile Ser Asp Lys Ile Lys Phe Leu Asn Pro Asp Arg Glu
            100                 105                 110
Tyr Asp Phe Arg Asp Leu Thr Trp Cys Ile Asn Pro Pro Glu Arg Ile
        115                 120                 125
Lys Leu Asp Tyr Asp Gln Tyr Cys Ala Asp Val Ala Ala Glu Glu Leu
    130                 135                 140
Met Asn Ala Leu Val Asn Ser Thr Leu Leu Glu Thr Arg Thr Thr Asn
145                 150                 155                 160
Gln Phe Leu Ala Val Ser Lys Gly Asn Cys Ser Gly Pro Thr Thr Ile
                165                 170                 175
Arg Gly Gln Phe Ser Asn Met Ser Leu Ser Leu Leu Asp Leu Tyr Leu
            180                 185                 190
Gly Arg Gly Tyr Asn Val Ser Ser Ile Val Thr Met Thr Ser Gln Gly
        195                 200                 205
Met Tyr Gly Gly Thr Tyr Leu Val Glu Lys Pro Asn Leu Ser Ser Lys
    210                 215                 220
Arg Ser Glu Leu Ser Gln Leu Ser Met Tyr Arg Val Phe Glu Val Gly
225                 230                 235                 240
Val Ile Arg Asn Pro Gly Leu Gly Ala Pro Val Phe His Met Thr Asn
                245                 250                 255
Tyr Leu Glu Gln Pro Val Ser Asn Asp Leu Ser Asn Cys Met Val Ala
            260                 265                 270
Leu Gly Glu Leu Lys Leu Ala Ala Leu Cys His Gly Glu Asp Ser Ile
        275                 280                 285
Thr Ile Pro Tyr Gln Gly Ser Gly Lys Gly Val Ser Phe Gln Leu Val
    290                 295                 300
Lys Leu Gly Val Trp Lys Ser Pro Thr Asp Met Gln Ser Trp Val Pro
305                 310                 315                 320
Leu Ser Thr Asp Asp Pro Val Ile Asp Arg Leu Tyr Leu Ser Ser His
                325                 330                 335
Arg Gly Val Ile Ala Asp Asn Gln Ala Lys Trp Ala Val Pro Thr Thr
            340                 345                 350
Arg Thr Asp Asp Lys Leu Arg Met Glu Thr Cys Phe Gln Gln Ala Cys
        355                 360                 365
Lys Gly Lys Ile Gln Ala Leu Cys Glu Asn Pro Glu Trp Ala Pro Leu
    370                 375                 380
Lys Asp Asn Arg Ile Pro Ser Tyr Gly Val Leu Ser Val Asp Leu Ser
385                 390                 395                 400
Leu Thr Val Glu Leu Lys Ile Lys Ile Ala Ser Gly Phe Gly Pro Leu
                405                 410                 415
Ile Thr His Gly Ser Gly Met Asp Leu Tyr Lys Ser Asn His Asn Asn
            420                 425                 430
Val Tyr Trp Leu Thr Ile Pro Pro Met Lys Asn Leu Ala Leu Gly Val
        435                 440                 445
Ile Asn Thr Leu Glu Trp Ile Pro Arg Phe Lys Val Ser Pro Ala Leu
    450                 455                 460
Phe Thr Val Pro Ile Lys Glu Ala Gly Gly Asp Cys His Ala Pro Thr
465                 470                 475                 480
Tyr Leu Pro Ala Glu Val Asp Gly Asp Val Lys Leu Ser Ser Asn Leu
                485                 490                 495
Val Ile Leu Pro Gly Gln Asp Leu Gln Tyr Val Leu Ala Thr Tyr Asp
            500                 505                 510
Thr Ser Ala Val Glu His Ala Val Val Tyr Tyr Val Tyr Ser Pro Ser
        515                 520                 525

Arg Leu Ser Ser Tyr Phe Tyr Pro Phe Arg Leu Pro Ile Lys Gly Val
    530                 535                 540
Pro Ile Glu Leu Gln Val Glu Cys Phe Thr Trp Asp Gln Lys Leu Trp
545                 550                 555                 560
Cys Arg His Phe Cys Val Leu Ala Asp Ser Glu Ser Gly Gly His Ile
                565                 570                 575
Thr His Ser Gly Met Val Gly Met Gly Val Ser Cys Thr Val Thr Arg
            580                 585                 590
Glu Asp Gly Thr Asn Gly Gly Gly Gly Ser Gly Gly Gly Gly Ser Gly
        595                 600                 605
Gly Gly Gly Ser Ala Ala Gln Pro Ala Glu Val Gln Leu Val Gln Ser
    610                 615                 620
Gly Ala Glu Leu Val Lys Pro Gly Ala Ser Val Lys Leu Ser Cys Lys
625                 630                 635                 640
Val Ser Asp Tyr Asn Ile Arg Arg Thr Tyr Met His Trp Val Arg Gln
                645                 650                 655
Arg Pro Gly Lys Gly Leu Glu Trp Ile Gly Arg Ile Asp Pro Ala Asn
            660                 665                 670
Gly Asn Thr Ile Tyr Gly Glu Lys Phe Lys Ser Lys Ala Thr Leu Thr
        675                 680                 685
Ala Asp Thr Ser Ser Asn Thr Ala Tyr Met Gln Leu Ser Gln Leu Lys
    690                 695                 700
Ser Asp Asp Thr Ala Ile Tyr Tyr Cys Ala Ile Gly Val Gln Tyr Leu
705                 710                 715                 720
Asp Tyr Trp Gly Gln Gly Thr Thr Val Thr Val Ser Ser Gly Gly Gly
                725                 730                 735
Gly Ser Gly Gly Gly Gly Ser Gly Gly Gly Gly Ser Asp Ile Val Leu
            740                 745                 750
Thr Gln Ser Pro Ala Leu Ala Val Ser Pro Gly Glu Arg Val Thr Ile
        755                 760                 765
Ser Cys Arg Ala Thr Glu Ser Val Ser Thr Leu Ile His Trp Tyr Gln
    770                 775                 780
Gln Arg Pro Gly Gln Gln Pro Lys Leu Leu Ile Tyr Leu Thr Ser His
785                 790                 795                 800
Leu Asp Ser Gly Val Pro Ala Arg Phe Ser Gly Ser Gly Ser Gly Thr
                805                 810                 815
Asp Phe Thr Leu Thr Ile Asp Pro Val Glu Ala Asp Asp Thr Ala Thr
            820                 825                 830
Tyr Tyr Cys Gln Gln Thr Trp Asn Asp Pro Trp Thr Phe Gly Gly Gly
        835                 840                 845
Thr Lys Leu Glu Leu Lys Arg Ala Ala Ala Arg Gly Ser His His His
    850                 855                 860
His His His
865

<210> 7
<211> 869
<212> PRT
<213> Artificial Sequence

<220>
<223> construct for anti- mouse CD8-scFv

<400> 7
Met Gly Ser Arg Ile Val Ile Asn Arg Glu His Leu Met Ile Asp Arg
1               5                   10                  15
Pro Tyr Val Leu Leu Ala Val Leu Phe Val Met Phe Leu Ser Leu Ile
            20                  25                  30
Gly Leu Leu Ala Ile Ala Gly Ile Arg Leu His Arg Ala Ala Ile Tyr
        35                  40                  45
Thr Ala Glu Ile His Lys Ser Leu Ser Thr Asn Leu Asp Val Thr Asn
    50                  55                  60
Ser Ile Glu His Gln Val Lys Asp Val Leu Thr Pro Leu Phe Lys Ile

65                  70                  75                  80
Ile Gly Asp Glu Val Gly Leu Arg Thr Pro Gln Arg Phe Thr Asp Leu
                85                  90                  95
Val Lys Phe Ile Ser Asp Lys Ile Lys Phe Leu Asn Pro Asp Arg Glu
            100                 105                 110
Tyr Asp Phe Arg Asp Leu Thr Trp Cys Ile Asn Pro Pro Glu Arg Ile
        115                 120                 125
Lys Leu Asp Tyr Asp Gln Tyr Cys Ala Asp Val Ala Ala Glu Glu Leu
    130                 135                 140
Met Asn Ala Leu Val Asn Ser Thr Leu Leu Glu Thr Arg Thr Thr Asn
145                 150                 155                 160
Gln Phe Leu Ala Val Ser Lys Gly Asn Cys Ser Gly Pro Thr Thr Ile
                165                 170                 175
Arg Gly Gln Phe Ser Asn Met Ser Leu Ser Leu Leu Asp Leu Tyr Leu
            180                 185                 190
Gly Arg Gly Tyr Asn Val Ser Ser Ile Val Thr Met Thr Ser Gln Gly
        195                 200                 205
Met Tyr Gly Gly Thr Tyr Leu Val Glu Lys Pro Asn Leu Ser Ser Lys
    210                 215                 220
Arg Ser Glu Leu Ser Gln Leu Ser Met Tyr Arg Val Phe Glu Val Gly
225                 230                 235                 240
Val Ile Arg Asn Pro Gly Leu Gly Ala Pro Val Phe His Met Thr Asn
                245                 250                 255
Tyr Leu Glu Gln Pro Val Ser Asn Asp Leu Ser Asn Cys Met Val Ala
            260                 265                 270
Leu Gly Glu Leu Lys Leu Ala Ala Leu Cys His Gly Glu Asp Ser Ile
        275                 280                 285
Thr Ile Pro Tyr Gln Gly Ser Gly Lys Gly Val Ser Phe Gln Leu Val
    290                 295                 300
Lys Leu Gly Val Trp Lys Ser Pro Thr Asp Met Gln Ser Trp Val Pro
305                 310                 315                 320
Leu Ser Thr Asp Asp Pro Val Ile Asp Arg Leu Tyr Leu Ser Ser His
                325                 330                 335
Arg Gly Val Ile Ala Asp Asn Gln Ala Lys Trp Ala Val Pro Thr Thr
            340                 345                 350
Arg Thr Asp Asp Lys Leu Arg Met Glu Thr Cys Phe Gln Gln Ala Cys
        355                 360                 365
Lys Gly Lys Ile Gln Ala Leu Cys Glu Asn Pro Glu Trp Ala Pro Leu
    370                 375                 380
Lys Asp Asn Arg Ile Pro Ser Tyr Gly Val Leu Ser Val Asp Leu Ser
385                 390                 395                 400
Leu Thr Val Glu Leu Lys Ile Lys Ile Ala Ser Gly Phe Gly Pro Leu
                405                 410                 415
Ile Thr His Gly Ser Gly Met Asp Leu Tyr Lys Ser Asn His Asn Asn
            420                 425                 430
Val Tyr Trp Leu Thr Ile Pro Pro Met Lys Asn Leu Ala Leu Gly Val
        435                 440                 445
Ile Asn Thr Leu Glu Trp Ile Pro Arg Phe Lys Val Ser Pro Ala Leu
    450                 455                 460
Phe Thr Val Pro Ile Lys Glu Ala Gly Gly Asp Cys His Ala Pro Thr
465                 470                 475                 480
Tyr Leu Pro Ala Glu Val Asp Gly Asp Val Lys Leu Ser Ser Asn Leu
                485                 490                 495
Val Ile Leu Pro Gly Gln Asp Leu Gln Tyr Val Leu Ala Thr Tyr Asp
            500                 505                 510
Thr Ser Ala Val Glu His Ala Val Val Tyr Tyr Val Tyr Ser Pro Ser
        515                 520                 525
Arg Leu Ser Ser Tyr Phe Tyr Pro Phe Arg Leu Pro Ile Lys Gly Val
    530                 535                 540
Pro Ile Glu Leu Gln Val Glu Cys Phe Thr Trp Asp Gln Lys Leu Trp
545                 550                 555                 560
Cys Arg His Phe Cys Val Leu Ala Asp Ser Glu Ser Gly Gly His Ile
                565                 570                 575

Thr His Ser Gly Met Val Gly Met Gly Val Ser Cys Thr Val Thr Arg
            580                 585                 590
Glu Asp Gly Thr Asn Gly Gly Gly Gly Ser Gly Gly Gly Gly Ser Gly
        595                 600                 605
Gly Gly Gly Ser Ala Ala Gln Pro Ala Glu Val Gln Leu Val Glu Ser
    610                 615                 620
Gly Gly Gly Leu Val Gln Pro Gly Arg Ser Leu Lys Leu Ser Cys Ala
625                 630                 635                 640
Ala Ser Gly Phe Thr Phe Ser Asn Tyr Tyr Met Ala Trp Val Arg Gln
                645                 650                 655
Ala Pro Thr Lys Gly Leu Glu Trp Val Ala Tyr Ile Asn Thr Gly Gly
            660                 665                 670
Gly Thr Thr Tyr Tyr Arg Asp Ser Val Lys Gly Arg Phe Thr Ile Ser
        675                 680                 685
Arg Asp Asp Ala Lys Ser Thr Leu Tyr Leu Gln Met Asp Ser Leu Arg
    690                 695                 700
Ser Glu Asp Thr Ala Thr Tyr Tyr Cys Thr Thr Ala Ile Gly Tyr Tyr
705                 710                 715                 720
Phe Asp Tyr Trp Gly Gln Gly Val Met Val Thr Val Ser Ser Gly Gly
                725                 730                 735
Gly Gly Ser Gly Gly Gly Gly Ser Gly Gly Gly Gly Ser Asp Ile Gln
            740                 745                 750
Met Thr Gln Ser Pro Ala Ser Leu Ser Ala Ser Leu Gly Glu Thr Val
        755                 760                 765
Ser Ile Glu Cys Leu Ala Ser Glu Asp Ile Tyr Ser Tyr Leu Ala Trp
    770                 775                 780
Tyr Gln Gln Lys Pro Gly Lys Ser Pro Gln Val Leu Ile Tyr Ala Ala
785                 790                 795                 800
Asn Arg Leu Gln Asp Gly Val Pro Ser Arg Phe Ser Gly Ser Gly Ser
                805                 810                 815
Gly Thr Gln Tyr Ser Leu Lys Ile Ser Gly Met Gln Pro Glu Asp Glu
            820                 825                 830
Gly Asp Tyr Phe Cys Leu Gln Gly Ser Lys Phe Pro Tyr Thr Phe Gly
        835                 840                 845
Ala Gly Thr Lys Leu Glu Leu Lys Arg Ala Ala Ala Arg Gly Ser His
    850                 855                 860
His His His His His
865

<210> 8
<211> 738
<212> PRT
<213> Artificial Sequence

<220>
<223> construct for anti-human CD4-DARPin

<400> 8
Met Gly Ser Arg Ile Val Ile Asn Arg Glu His Leu Met Ile Asp Arg
1               5                   10                  15
Pro Tyr Val Leu Leu Ala Val Leu Phe Val Met Phe Leu Ser Leu Ile
            20                  25                  30
Gly Leu Leu Ala Ile Ala Gly Ile Arg Leu His Arg Ala Ala Ile Tyr
        35                  40                  45
Thr Ala Glu Ile His Lys Ser Leu Ser Thr Asn Leu Asp Val Thr Asn
    50                  55                  60
Ser Ile Glu His Gln Val Lys Asp Val Leu Thr Pro Leu Phe Lys Ile
65                  70                  75                  80
Ile Gly Asp Glu Val Gly Leu Arg Thr Pro Gln Arg Phe Thr Asp Leu
                85                  90                  95
Val Lys Phe Ile Ser Asp Lys Ile Lys Phe Leu Asn Pro Asp Arg Glu
            100                 105                 110
Tyr Asp Phe Arg Asp Leu Thr Trp Cys Ile Asn Pro Pro Glu Arg Ile

        115                 120                 125
Lys Leu Asp Tyr Asp Gln Tyr Cys Ala Asp Val Ala Ala Glu Glu Leu
    130                 135                 140
Met Asn Ala Leu Val Asn Ser Thr Leu Leu Glu Thr Arg Thr Thr Asn
145                 150                 155                 160
Gln Phe Leu Ala Val Ser Lys Gly Asn Cys Ser Gly Pro Thr Thr Ile
                165                 170                 175
Arg Gly Gln Phe Ser Asn Met Ser Leu Ser Leu Leu Asp Leu Tyr Leu
            180                 185                 190
Gly Arg Gly Tyr Asn Val Ser Ser Ile Val Thr Met Thr Ser Gln Gly
        195                 200                 205
Met Tyr Gly Gly Thr Tyr Leu Val Glu Lys Pro Asn Leu Ser Ser Lys
    210                 215                 220
Arg Ser Glu Leu Ser Gln Leu Ser Met Tyr Arg Val Phe Glu Val Gly
225                 230                 235                 240
Val Ile Arg Asn Pro Gly Leu Gly Ala Pro Val Phe His Met Thr Asn
                245                 250                 255
Tyr Leu Glu Gln Pro Val Ser Asn Asp Leu Ser Asn Cys Met Val Ala
            260                 265                 270
Leu Gly Glu Leu Lys Leu Ala Ala Leu Cys His Gly Glu Asp Ser Ile
        275                 280                 285
Thr Ile Pro Tyr Gln Gly Ser Gly Lys Gly Val Ser Phe Gln Leu Val
    290                 295                 300
Lys Leu Gly Val Trp Lys Ser Pro Thr Asp Met Gln Ser Trp Val Pro
305                 310                 315                 320
Leu Ser Thr Asp Asp Pro Val Ile Asp Arg Leu Tyr Leu Ser Ser His
                325                 330                 335
Arg Gly Val Ile Ala Asp Asn Gln Ala Lys Trp Ala Val Pro Thr Thr
            340                 345                 350
Arg Thr Asp Asp Lys Leu Arg Met Glu Thr Cys Phe Gln Gln Ala Cys
        355                 360                 365
Lys Gly Lys Ile Gln Ala Leu Cys Glu Asn Pro Glu Trp Ala Pro Leu
    370                 375                 380
Lys Asp Asn Arg Ile Pro Ser Tyr Gly Val Leu Ser Val Asp Leu Ser
385                 390                 395                 400
Leu Thr Val Glu Leu Lys Ile Lys Ile Ala Ser Gly Phe Gly Pro Leu
                405                 410                 415
Ile Thr His Gly Ser Gly Met Asp Leu Tyr Lys Ser Asn His Asn Asn
            420                 425                 430
Val Tyr Trp Leu Thr Ile Pro Pro Met Lys Asn Leu Ala Leu Gly Val
        435                 440                 445
Ile Asn Thr Leu Glu Trp Ile Pro Arg Phe Lys Val Ser Pro Ala Leu
    450                 455                 460
Phe Thr Val Pro Ile Lys Glu Ala Gly Gly Asp Cys His Ala Pro Thr
465                 470                 475                 480
Tyr Leu Pro Ala Glu Val Asp Gly Asp Val Lys Leu Ser Ser Asn Leu
                485                 490                 495
Val Ile Leu Pro Gly Gln Asp Leu Gln Tyr Val Leu Ala Thr Tyr Asp
            500                 505                 510
Thr Ser Ala Val Glu His Ala Val Val Tyr Tyr Val Tyr Ser Pro Ser
        515                 520                 525
Arg Leu Ser Ser Tyr Phe Tyr Pro Phe Arg Leu Pro Ile Lys Gly Val
    530                 535                 540
Pro Ile Glu Leu Gln Val Glu Cys Phe Thr Trp Asp Gln Lys Leu Trp
545                 550                 555                 560
Cys Arg His Phe Cys Val Leu Ala Asp Ser Glu Ser Gly Gly His Ile
                565                 570                 575
Thr His Ser Gly Met Val Gly Met Gly Val Ser Cys Thr Val Thr Arg
            580                 585                 590
Glu Asp Gly Thr Asn Ala Ala Gln Pro Ala Asp Leu Gly Lys Lys Leu
        595                 600                 605
Leu Glu Ala Ala Arg Ala Gly Gln Asp Asp Glu Val Arg Ile Leu Met
    610                 615                 620

Ala Asn Gly Ala Asp Val Asn Ala Thr Asp Thr Leu Gly Arg Thr Pro
625                 630                 635                 640
Leu His Leu Ala Ala Gln Asn Gly His Leu Glu Ile Val Glu Val Leu
                645                 650                 655
Leu Lys His Ser Ala Asp Val Asn Ala Ile Glu Glu Val Gly Met Thr
            660                 665                 670
Pro Leu His Leu Ala Val Val Ala Gly His Leu Glu Ile Val Glu Val
        675                 680                 685
Leu Leu Lys Asn Gly Ala Asp Val Asn Ala Gln Asp Lys Phe Gly Lys
    690                 695                 700
Thr Ala Phe Asp Ile Ser Ile Asp Tyr Gly Asn Glu Asp Leu Ala Glu
705                 710                 715                 720
Ile Leu Gln Lys Leu Asn Ala Ala Ala Arg Gly Ser His His His His
                725                 730                 735
His His

<210> 9
<211> 870
<212> PRT
<213> Artificial Sequence

<220>
<223> construct for anti- human CD8-scFv

<400> 9
Met Gly Ser Arg Ile Val Ile Asn Arg Glu His Leu Met Ile Asp Arg
1               5                   10                  15
Pro Tyr Val Leu Leu Ala Val Leu Phe Val Met Phe Leu Ser Leu Ile
            20                  25                  30
Gly Leu Leu Ala Ile Ala Gly Ile Arg Leu His Arg Ala Ala Ile Tyr
        35                  40                  45
Thr Ala Glu Ile His Lys Ser Leu Ser Thr Asn Leu Asp Val Thr Asn
    50                  55                  60
Ser Ile Glu His Gln Val Lys Asp Val Leu Thr Pro Leu Phe Lys Ile
65                  70                  75                  80
Ile Gly Asp Glu Val Gly Leu Arg Thr Pro Gln Arg Phe Thr Asp Leu
                85                  90                  95
Val Lys Phe Ile Ser Asp Lys Ile Lys Phe Leu Asn Pro Asp Arg Glu
            100                 105                 110
Tyr Asp Phe Arg Asp Leu Thr Trp Cys Ile Asn Pro Pro Glu Arg Ile
        115                 120                 125
Lys Leu Asp Tyr Asp Gln Tyr Cys Ala Asp Val Ala Ala Glu Glu Leu
    130                 135                 140
Met Asn Ala Leu Val Asn Ser Thr Leu Leu Glu Thr Arg Thr Thr Asn
145                 150                 155                 160
Gln Phe Leu Ala Val Ser Lys Gly Asn Cys Ser Gly Pro Thr Thr Ile
                165                 170                 175
Arg Gly Gln Phe Ser Asn Met Ser Leu Ser Leu Leu Asp Leu Tyr Leu
            180                 185                 190
Gly Arg Gly Tyr Asn Val Ser Ser Ile Val Thr Met Thr Ser Gln Gly
        195                 200                 205
Met Tyr Gly Gly Thr Tyr Leu Val Glu Lys Pro Asn Leu Ser Ser Lys
    210                 215                 220
Arg Ser Glu Leu Ser Gln Leu Ser Met Tyr Arg Val Phe Glu Val Gly
225                 230                 235                 240
Val Ile Arg Asn Pro Gly Leu Gly Ala Pro Val Phe His Met Thr Asn
                245                 250                 255
Tyr Leu Glu Gln Pro Val Ser Asn Asp Leu Ser Asn Cys Met Val Ala
            260                 265                 270
Leu Gly Glu Leu Lys Leu Ala Ala Leu Cys His Gly Glu Asp Ser Ile
        275                 280                 285
Thr Ile Pro Tyr Gln Gly Ser Gly Lys Gly Val Ser Phe Gln Leu Val

290                 295                 300
Lys Leu Gly Val Trp Lys Ser Pro Thr Asp Met Gln Ser Trp Val Pro
305                 310                 315                 320
Leu Ser Thr Asp Asp Pro Val Ile Asp Arg Leu Tyr Leu Ser Ser His
                325                 330                 335
Arg Gly Val Ile Ala Asp Asn Gln Ala Lys Trp Ala Val Pro Thr Thr
            340                 345                 350
Arg Thr Asp Asp Lys Leu Arg Met Glu Thr Cys Phe Gln Gln Ala Cys
        355                 360                 365
Lys Gly Lys Ile Gln Ala Leu Cys Glu Asn Pro Glu Trp Ala Pro Leu
    370                 375                 380
Lys Asp Asn Arg Ile Pro Ser Tyr Gly Val Leu Ser Val Asp Leu Ser
385                 390                 395                 400
Leu Thr Val Glu Leu Lys Ile Lys Ile Ala Ser Gly Phe Gly Pro Leu
                405                 410                 415
Ile Thr His Gly Ser Gly Met Asp Leu Tyr Lys Ser Asn His Asn Asn
            420                 425                 430
Val Tyr Trp Leu Thr Ile Pro Pro Met Lys Asn Leu Ala Leu Gly Val
        435                 440                 445
Ile Asn Thr Leu Glu Trp Ile Pro Arg Phe Lys Val Ser Pro Ala Leu
    450                 455                 460
Phe Thr Val Pro Ile Lys Glu Ala Gly Gly Asp Cys His Ala Pro Thr
465                 470                 475                 480
Tyr Leu Pro Ala Glu Val Asp Gly Asp Val Lys Leu Ser Ser Asn Leu
                485                 490                 495
Val Ile Leu Pro Gly Gln Asp Leu Gln Tyr Val Leu Ala Thr Tyr Asp
            500                 505                 510
Thr Ser Ala Val Glu His Ala Val Val Tyr Tyr Val Tyr Ser Pro Ser
        515                 520                 525
Arg Leu Ser Ser Tyr Phe Tyr Pro Phe Arg Leu Pro Ile Lys Gly Val
    530                 535                 540
Pro Ile Glu Leu Gln Val Glu Cys Phe Thr Trp Asp Gln Lys Leu Trp
545                 550                 555                 560
Cys Arg His Phe Cys Val Leu Ala Asp Ser Glu Ser Gly Gly His Ile
                565                 570                 575
Thr His Ser Gly Met Val Gly Met Gly Val Ser Cys Thr Val Thr Arg
            580                 585                 590
Glu Asp Gly Thr Asn Gly Gly Gly Gly Ser Gly Gly Gly Gly Ser Gly
        595                 600                 605
Gly Gly Gly Ser Ala Ala Gln Pro Ala Gln Val Gln Leu Val Gln Ser
    610                 615                 620
Gly Ala Glu Asp Lys Lys Pro Gly Ala Ser Val Lys Val Ser Cys Lys
625                 630                 635                 640
Ala Ser Gly Phe Asn Ile Lys Asp Thr Tyr Ile His Trp Val Arg Gln
                645                 650                 655
Ala Pro Gly Gln Gly Leu Glu Trp Met Gly Arg Ile Asp Pro Ala Asn
            660                 665                 670
Asp Asn Thr Leu Tyr Ala Ser Lys Phe Gln Gly Arg Val Thr Ile Thr
        675                 680                 685
Ala Asp Thr Ser Ser Asn Thr Ala Tyr Met Glu Leu Ser Ser Leu Arg
    690                 695                 700
Ser Glu Asp Thr Ala Val Tyr Tyr Cys Gly Arg Gly Tyr Gly Tyr Tyr
705                 710                 715                 720
Val Phe Asp His Trp Gly Gln Gly Thr Thr Val Thr Val Ser Ser Gly
                725                 730                 735
Gly Gly Gly Ser Gly Gly Gly Gly Ser Gly Gly Gly Gly Ser Asp Ile
            740                 745                 750
Val Met Thr Gln Ser Pro Ser Ser Leu Ser Ala Ser Val Gly Asp Arg
        755                 760                 765
Val Thr Ile Thr Cys Arg Thr Ser Arg Ser Ile Ser Gln Tyr Leu Ala
    770                 775                 780
Trp Tyr Gln Glu Lys Pro Gly Lys Ala Pro Lys Leu Leu Ile Tyr Ser
785                 790                 795                 800

Gly Ser Thr Leu Gln Ser Gly Val Pro Ser Arg Phe Ser Gly Ser Gly
                805                 810                 815
Ser Gly Thr Asp Phe Thr Leu Thr Ile Ser Ser Leu Gln Pro Glu Asp
            820                 825                 830
Phe Ala Thr Tyr Tyr Cys Gln Gln His Asn Glu Asn Pro Leu Thr Phe
        835                 840                 845
Gly Gln Gly Thr Lys Val Glu Ile Lys Arg Ala Ala Ala Arg Gly Ser
    850                 855                 860
His His His His His His
865                 870

<210> 10
<211> 523
<212> PRT
<213> Artificial Sequence

<220>
<223> MeV derived F protein Fcd30

<400> 10
Met Ser Ile Met Gly Leu Lys Val Asn Val Ser Ala Ile Phe Met Ala
1               5                   10                  15
Val Leu Leu Thr Leu Gln Thr Pro Thr Gly Gln Ile His Trp Gly Asn
            20                  25                  30
Leu Ser Lys Ile Gly Val Val Gly Ile Gly Ser Ala Ser Tyr Lys Val
        35                  40                  45
Met Thr Arg Ser Ser His Gln Ser Leu Val Ile Lys Leu Met Pro Asn
    50                  55                  60
Ile Thr Leu Leu Asn Asn Cys Thr Arg Val Glu Ile Ala Glu Tyr Arg
65                  70                  75                  80
Arg Leu Leu Arg Thr Val Leu Glu Pro Ile Arg Asp Ala Leu Asn Ala
                85                  90                  95
Met Thr Gln Asn Ile Arg Pro Val Gln Ser Val Ala Ser Ser Arg Arg
            100                 105                 110
His Lys Arg Phe Ala Gly Val Val Leu Ala Gly Ala Ala Leu Gly Val
        115                 120                 125
Ala Thr Ala Ala Gln Ile Thr Ala Gly Ile Ala Leu His Gln Ser Met
    130                 135                 140
Leu Asn Ser Gln Ala Ile Asp Asn Leu Arg Ala Ser Leu Glu Thr Thr
145                 150                 155                 160
Asn Gln Ala Ile Glu Ala Ile Arg Gln Ala Gly Gln Glu Met Ile Leu
                165                 170                 175
Ala Val Gln Gly Val Gln Asp Tyr Ile Asn Asn Glu Leu Ile Pro Ser
            180                 185                 190
Met Asn Gln Leu Ser Cys Asp Leu Ile Gly Gln Lys Leu Gly Leu Lys
        195                 200                 205
Leu Leu Arg Tyr Tyr Thr Glu Ile Leu Ser Leu Phe Gly Pro Ser Leu
    210                 215                 220
Arg Asp Pro Ile Ser Ala Glu Ile Ser Ile Gln Ala Leu Ser Tyr Ala
225                 230                 235                 240
Leu Gly Gly Asp Ile Asn Lys Val Leu Glu Lys Leu Gly Tyr Ser Gly
                245                 250                 255
Gly Asp Leu Leu Gly Ile Leu Glu Ser Arg Gly Ile Lys Ala Arg Ile
            260                 265                 270
Thr His Val Asp Thr Glu Ser Tyr Phe Ile Val Leu Ser Ile Ala Tyr
        275                 280                 285
Pro Thr Leu Ser Glu Ile Lys Gly Val Ile Val His Arg Leu Glu Gly
    290                 295                 300
Val Ser Tyr Asn Ile Gly Ser Gln Glu Trp Tyr Thr Thr Val Pro Lys
305                 310                 315                 320
Tyr Val Ala Thr Gln Gly Tyr Leu Ile Ser Asn Phe Asp Glu Ser Ser
                325                 330                 335
Cys Thr Phe Met Pro Glu Gly Thr Val Cys Ser Gln Asn Ala Leu Tyr

340                 345                 350
Pro Met Ser Pro Leu Leu Gln Glu Cys Leu Arg Gly Ser Thr Lys Ser
        355                 360                 365
Cys Ala Arg Thr Leu Val Ser Gly Ser Phe Gly Asn Arg Phe Ile Leu
    370                 375                 380
Ser Gln Gly Asn Leu Ile Ala Asn Cys Ala Ser Ile Leu Cys Lys Cys
385                 390                 395                 400
Tyr Thr Thr Gly Thr Ile Ile Asn Gln Asp Pro Asp Lys Ile Leu Thr
                405                 410                 415
Tyr Ile Ala Ala Asp His Cys Pro Val Val Glu Val Asn Gly Val Thr
            420                 425                 430
Ile Gln Val Gly Ser Arg Arg Tyr Pro Asp Ala Val Tyr Leu His Arg
        435                 440                 445
Ile Asp Leu Gly Pro Pro Ile Ser Leu Glu Arg Leu Asp Val Gly Thr
    450                 455                 460
Asn Leu Gly Asn Ala Ile Ala Lys Leu Glu Asp Ala Lys Glu Leu Leu
465                 470                 475                 480
Glu Ser Ser Asp Gln Ile Leu Arg Ser Met Lys Gly Leu Ser Ser Thr
                485                 490                 495
Ser Ile Val Tyr Ile Leu Ile Ala Val Cys Leu Gly Gly Leu Ile Gly
            500                 505                 510
Ile Pro Ala Leu Ile Cys Cys Cys Arg Gly Arg
        515                 520

<210> 11
<211> 3499
<212> DNA
<213> Artificial Sequence

<220>
<223> SB-MP71-hCD4-P2A-mBcl-xl

<400> 11
cagttgaagt cggaagttta catacactta agttggagtc attaaaactc gtttttcaac      60

tactccacaa atttcttgtt aacaaacaat agttttggca agtcagttag gacatctact     120

ttgtgcatga cacaagtcat ttttccaaca attgtttaca gacagattat ttcacttata     180

attcactgta tcacaattcc agtgggtcag aagtttacat acactaagtt gactgtgcct     240

ttaaacagct tggaaaattc cagaaaatga tgtcatggct ttagaagctt gagaagttca     300

gatcaaggtt aggaacagag agacagcaga atatgggcca aacaggatat ctgtggtaag     360

cagttcctgc cccggctcag ggccaagaac agttggaaca gcagaatatg ggccaaacag     420

gatatctgtg gtaagcagtt cctgccccgg ctcagggcca agaacagatg gtccccagat     480

gcggtcccgc cctcagcagt ttctagagaa ccatcagatg tttccagggt gccccaagga     540

cctgaaatga ccctgtgcct tatttgaact aaccaatcag ttcgcttctc gcttctgttc     600

gcgcgcttct gctccccgag ctcaataaaa gagcccacaa cccctcactc ggcgcgccag     660

tcctccgata gactgcgtcg cccgggtacc cgtgttctca ataaaccctc ttgcagttgc     720

atccgactcg tggtctcgct gttccttggg agggtctcct ctgagtgatt gactgcccac     780

ctcgggggtc tttcatttgt ccggagaggc actgggcagg taagtatcaa ggttacaaga     840

caggtttaag gagaccaata gaaactgggc ttgtcgagac agaacgcgta attaatgcat     900

gaagactctt gcgtttctga taggcaccta ttggtcttac tgacatccac tttgcctttc 960
tctccacagg tgtccactcc cagcggccgc catgaaccgg ggagtccctt ttaggcactt 1020
gcttctggtg ctgcaactgg cgctcctccc agcagccact cagggaaaga aagtggtgct 1080
gggcaaaaaa ggggatacag tggaactgac ctgtacagct tcccagaaga agagcataca 1140
attccactgg aaaaactcca accagataaa gattctggga aatcagggct ccttcttaac 1200
taaaggtcca tccaagctga atgatcgcgc tgactcaaga agaagccttt gggaccaagg 1260
aaactttccc ctgatcatca agaatcttaa gatagaagac tcagatactt acatctgtga 1320
agtggaggac cagaaggagg aggtgcaatt gctagtgttc ggattgactg ccaactctga 1380
cacccacctg cttcaggggc agagcctgac cctgaccttg gagagccccc ctggtagtag 1440
cccctcagtg caatgtagga gtccaagggg taaaaacata caggggggga agaccctctc 1500
cgtgtctcag ctggagctcc aggatagtgg cacctggaca tgcactgtct tgcagaacca 1560
gaagaaggtg gagttcaaaa tagacatcgt ggtgctagct ttccagaagg cctccagcat 1620
agtctataag aaagaggggg aacaggtgga gttctccttc ccactcgcct ttacagttga 1680
aaagctgacg ggcagtggcg agctgtggtg gcaggcggag agggcttcct cctccaagtc 1740
ttggatcacc tttgacctga agaacaagga agtgtctgta aaacgggtta cccaggaccc 1800
taagctccag atgggcaaga agctcccgct ccacctcacc ctgccccagg ccttgcctca 1860
gtatgctggc tctggaaacc tcaccctggc ccttgaagcg aaaacaggaa agttgcatca 1920
ggaagtgaac ctggtggtga tgagagccac tcagctccag aaaaatttga cctgtgaggt 1980
gtggggaccc acctccccta agctgatgct gagtttgaaa ctggagaaca aggaggcaaa 2040
ggtctcgaag cgggagaagg cggtgtgggt gctgaaccct gaggcgggga tgtggcagtg 2100
tctgctgagt gactcgggac aggtcctgct ggaatccaac atcaaggttc tgcccacatg 2160
gtccaccccg gtgcagccaa tggccctgat tgtgctgggg ggcgtcgccg gcctcctgct 2220
tttcattggg ctaggcatct tcttctgtgt caggtgccgg caccgaaggc gccaagcaga 2280
gcggatgtct cagatcaaga gactcctcag tgagaagaag acctgccagt gtcctcaccg 2340
gtttcagaag acatgtagcc ccattggcag cggcgccacc aacttcagcc tgctgaagca 2400
ggccggcgac gtggaagaga accccggccc catgtctcag agcaaccggg agctggtggt 2460
cgactttctc tcctacaagc tttcccagaa aggatacagc tggagtcagt ttagtgatgt 2520
cgaagagaat aggactgagg ccccagaaga aactgaagca gagagggaga cccccagtgc 2580
catcaatggc aacccatcct ggcacctggc ggatagcccg gccgtgaatg gagccactgg 2640
ccacagcagc agtttggatg cgcgggaggt gattcccatg gcagcagtga agcaagcgct 2700
gagagaggca ggcgatgagt ttgaactgcg gtaccggaga gcgttcagtg atctaacatc 2760
ccagcttcac ataaccccag ggaccgcgta tcagagcttt gagcaggtag tgaatgaact 2820

ctttcgggat ggagtaaact ggggtcgcat cgtggccttt ttctcctttg gcggggcact 2880

gtgcgtggaa agcgtagaca aggagatgca ggtattggtg agtcggattg caagttggat 2940

ggccacctat ctgaatgacc acctagagcc ttggatccag gagaacggcg gctgggacac 3000

ttttgtggat ctctacggga acaatgcagc agccgagagc cggaaaggcc aggagcgctt 3060

caaccgctgg ttcctgacgg gcatgactgt ggctggtgtg gttctgctgg gctcactctt 3120

cagtcggaag tgagaattca ataaaatatc tttattttca ttacatctgt gtgttggttt 3180

tttgtgtgta attcggatct agcttgtgga aggctactcg aaatgtttga cccaagttaa 3240

acaatttaaa ggcaatgcta ccaaatacta attgagtgta tgtaaacttc tgacccactg 3300

ggaatgtgat gaaagaaata aaagctgaaa tgaatcattc tctctactat tattctgata 3360

tttcacattc ttaaaataaa gtggtgatcc taactgacct aagacaggga atttttacta 3420

ggattaaatg tcaggaattg tgaaaaagtg agtttaaatg tatttggcta aggtgtatgt 3480

aaacttccga cttcaactg 3499

<210> 12
<211> 2830
<212> DNA
<213> Artificial Sequence

<220>
<223> SB-MP71-hCD8a-P2A-mBcl-xl

<400> 12
cagttgaagt cggaagttta catacactta agttggagtc attaaaactc gtttttcaac 60

tactccacaa atttcttgtt aacaaacaat agttttggca agtcagttag gacatctact 120

ttgtgcatga cacaagtcat ttttccaaca attgtttaca gacagattat ttcacttata 180

attcactgta tcacaattcc agtgggtcag aagtttacat acactaagtt gactgtgcct 240

ttaaacagct tggaaaattc cagaaaatga tgtcatggct ttagaagctt gagaagttca 300

gatcaaggtt aggaacagag agacagcaga atatgggcca aacaggatat ctgtggtaag 360

cagttcctgc cccggctcag ggccaagaac agttggaaca gcagaatatg ggccaaacag 420

gatatctgtg gtaagcagtt cctgccccgg ctcagggcca agaacagatg gtccccagat 480

gcggtcccgc cctcagcagt ttctagagaa ccatcagatg tttccagggt gccccaagga 540

cctgaaatga ccctgtgcct tatttgaact aaccaatcag ttcgcttctc gcttctgttc 600

gcgcgcttct gctccccgag ctcaataaaa gagcccacaa cccctcactc ggcgcgccag 660

tcctccgata gactgcgtcg cccgggtacc cgtgttctca ataaaccctc ttgcagttgc 720

atccgactcg tggtctcgct gttccttggg agggtctcct ctgagtgatt gactgcccac 780

ctcgggggtc tttcatttgt ccggagaggc actgggcagg taagtatcaa ggttacaaga 840

caggtttaag gagaccaata gaaactgggc ttgtcgagac agaacgcgta attaatgcat 900

gaagactctt gcgtttctga taggcaccta ttggtcttac tgacatccac tttgcctttc 960

tctccacagg tgtccactcc cagcggccgc catggcctta ccagtgaccg ccttgctcct 1020

gccgctggcc ttgctgctcc acgccgccag gccgagccag ttccgggtgt cgccgctgga 1080

tcggacctgg aacctgggcg agacagtgga gctgaagtgc caggtgctgc tgtccaaccc 1140

gacgtcgggc tgctcgtggc tcttccagcc gcgcggcgcc gccgccagtc ccaccttcct 1200

cctatacctc tcccaaaaca agcccaaggc ggccgagggg ctggacaccc agcggttctc 1260

gggcaagagg ttgggggaca ccttcgtcct caccctgagc gacttccgcc gagagaacga 1320

gggctactat ttctgctcgg ccctgagcaa ctccatcatg tacttcagcc acttcgtgcc 1380

ggtcttcctg ccagcgaagc ccaccacgac gccagcgccg cgaccaccaa caccggcgcc 1440

caccatcgcg tcgcagcccc tgtccctgcg cccagaggcg tgccggccag cggcgggggg 1500

cgcagtgcac acgagggggc tggacttcgc ctgtgatatc tacatctggg cgcccttggc 1560

cgggacttgt ggggtccttc tcctgtcact ggttatcacc ctttactgca accacaggaa 1620

ccgaagacgt gtttgcaaat gtccccggcc tgtggtcaaa tcgggagaca agcccagcct 1680

ttcggcgaga tacgtcggca gcggcgccac caacttcagc ctgctgaagc aggccggcga 1740

cgtggaagag aaccccgggc ccatgtctca gagcaaccgg gagctggtgg tcgactttct 1800

ctcctacaag ctttcccaga aaggatacag ctggagtcag tttagtgatg tcgaagagaa 1860

taggactgag gccccagaag aaactgaagc agagagggag acccccagtg ccatcaatgg 1920

caacccatcc tggcacctgg cggatagccc ggccgtgaat ggagccactg gccacagcag 1980

cagtttggat gcgcgggagg tgattcccat ggcagcagtg aagcaagcgc tgagagaggc 2040

aggcgatgag tttgaactgc ggtaccggag agcgttcagt gatctaacat cccagcttca 2100

cataacccca gggaccgcgt atcagagctt tgagcaggta gtgaatgaac tctttcggga 2160

tggagtaaac tggggtcgca tcgtggcctt tttctccttt ggcggggcac tgtgcgtgga 2220

aagcgtagac aaggagatgc aggtattggt gagtcggatt gcaagttgga tggccaccta 2280

tctgaatgac cacctagagc cttggatcca ggagaacggc ggctgggaca cttttgtgga 2340

tctctacggg aacaatgcag cagccgagag ccggaaaggc caggagcgct tcaaccgctg 2400

gttcctgacg ggcatgactg tggctggtgt ggttctgctg ggctcactct tcagtcggaa 2460

gtgagaattc aataaaatat ctttattttc attacatctg tgtgttggtt ttttgtgtgt 2520

aattcggatc tagcttgtgg aaggctactc gaaatgtttg acccaagtta aacaatttaa 2580

aggcaatgct accaaatact aattgagtgt atgtaaactt ctgacccact gggaatgtga 2640

tgaaagaaat aaaagctgaa atgaatcatt ctctctacta ttattctgat atttcacatt 2700

cttaaaataa agtggtgatc ctaactgacc taagacaggg aatttttact aggattaaat 2760

gtcaggaatt gtgaaaaagt gagtttaaat gtatttggct aaggtgtatg taaacttccg    2820

acttcaactg                                                          2830

<210> 13
<211> 162
<212> PRT
<213> Mus musculus

<220>
<223> mCD90.2

<400> 13
Met Asn Pro Ala Ile Ser Val Ala Leu Leu Leu Ser Val Leu Gln Val
1               5                   10                  15
Ser Arg Gly Gln Lys Val Thr Ser Leu Thr Ala Cys Leu Val Asn Gln
            20                  25                  30
Asn Leu Arg Leu Asp Cys Arg His Glu Asn Asn Thr Lys Asp Asn Ser
        35                  40                  45
Ile Gln His Glu Phe Ser Leu Thr Arg Glu Lys Arg Lys His Val Leu
    50                  55                  60
Ser Gly Thr Leu Gly Ile Pro Glu His Thr Tyr Arg Ser Arg Val Thr
65                  70                  75                  80
Leu Ser Asn Gln Pro Tyr Ile Lys Val Leu Thr Leu Ala Asn Phe Thr
                85                  90                  95
Thr Lys Asp Glu Gly Asp Tyr Phe Cys Glu Leu Gln Val Ser Gly Ala
            100                 105                 110
Asn Pro Met Ser Ser Asn Lys Ser Ile Ser Val Tyr Arg Asp Lys Leu
        115                 120                 125
Val Lys Cys Gly Gly Ile Ser Leu Leu Val Gln Asn Thr Ser Trp Met
    130                 135                 140
Leu Leu Leu Leu Leu Ser Leu Ser Leu Leu Gln Ala Leu Asp Phe Ile
145                 150                 155                 160
Ser Leu

<210> 14
<211> 233
<212> PRT
<213> Mus musculus

<220>
<223> mBcl-xl

<400> 14
Met Ser Gln Ser Asn Arg Glu Leu Val Val Asp Phe Leu Ser Tyr Lys
1               5                   10                  15
Leu Ser Gln Lys Gly Tyr Ser Trp Ser Gln Phe Ser Asp Val Glu Glu
            20                  25                  30
Asn Arg Thr Glu Ala Pro Glu Glu Thr Glu Ala Glu Arg Glu Thr Pro
        35                  40                  45
Ser Ala Ile Asn Gly Asn Pro Ser Trp His Leu Ala Asp Ser Pro Ala
    50                  55                  60
Val Asn Gly Ala Thr Gly His Ser Ser Ser Leu Asp Ala Arg Glu Val
65                  70                  75                  80
Ile Pro Met Ala Ala Val Lys Gln Ala Leu Arg Glu Ala Gly Asp Glu
                85                  90                  95
Phe Glu Leu Arg Tyr Arg Arg Ala Phe Ser Asp Leu Thr Ser Gln Leu
            100                 105                 110
His Ile Thr Pro Gly Thr Ala Tyr Gln Ser Phe Glu Gln Val Val Asn
        115                 120                 125

Glu Leu Phe Arg Asp Gly Val Asn Trp Gly Arg Ile Val Ala Phe Phe
    130                 135                 140
Ser Phe Gly Gly Ala Leu Cys Val Glu Ser Val Asp Lys Glu Met Gln
145                 150                 155                 160
Val Leu Val Ser Arg Ile Ala Ser Trp Met Ala Thr Tyr Leu Asn Asp
                165                 170                 175
His Leu Glu Pro Trp Ile Gln Glu Asn Gly Gly Trp Asp Thr Phe Val
            180                 185                 190
Asp Leu Tyr Gly Asn Asn Ala Ala Ala Glu Ser Arg Lys Gly Gln Glu
        195                 200                 205
Arg Phe Asn Arg Trp Phe Leu Thr Gly Met Thr Val Ala Gly Val Val
    210                 215                 220
Leu Leu Gly Ser Leu Phe Ser Arg Lys
225                 230

<210> 15
<211> 458
<212> PRT
<213> Homo sapiens

<220>
<223> hCD4

<400> 15
Met Asn Arg Gly Val Pro Phe Arg His Leu Leu Leu Val Leu Gln Leu
1               5                   10                  15
Ala Leu Leu Pro Ala Ala Thr Gln Gly Lys Lys Val Val Leu Gly Lys
            20                  25                  30
Lys Gly Asp Thr Val Glu Leu Thr Cys Thr Ala Ser Gln Lys Lys Ser
        35                  40                  45
Ile Gln Phe His Trp Lys Asn Ser Asn Gln Ile Lys Ile Leu Gly Asn
    50                  55                  60
Gln Gly Ser Phe Leu Thr Lys Gly Pro Ser Lys Leu Asn Asp Arg Ala
65                  70                  75                  80
Asp Ser Arg Arg Ser Leu Trp Asp Gln Gly Asn Phe Pro Leu Ile Ile
                85                  90                  95
Lys Asn Leu Lys Ile Glu Asp Ser Asp Thr Tyr Ile Cys Glu Val Glu
            100                 105                 110
Asp Gln Lys Glu Glu Val Gln Leu Leu Val Phe Gly Leu Thr Ala Asn
        115                 120                 125
Ser Asp Thr His Leu Leu Gln Gly Gln Ser Leu Thr Leu Thr Leu Glu
    130                 135                 140
Ser Pro Pro Gly Ser Ser Pro Ser Val Gln Cys Arg Ser Pro Arg Gly
145                 150                 155                 160
Lys Asn Ile Gln Gly Gly Lys Thr Leu Ser Val Ser Gln Leu Glu Leu
                165                 170                 175
Gln Asp Ser Gly Thr Trp Thr Cys Thr Val Leu Gln Asn Gln Lys Lys
            180                 185                 190
Val Glu Phe Lys Ile Asp Ile Val Val Leu Ala Phe Gln Lys Ala Ser
        195                 200                 205
Ser Ile Val Tyr Lys Lys Glu Gly Glu Gln Val Glu Phe Ser Phe Pro
    210                 215                 220
Leu Ala Phe Thr Val Glu Lys Leu Thr Gly Ser Gly Glu Leu Trp Trp
225                 230                 235                 240
Gln Ala Glu Arg Ala Ser Ser Ser Lys Ser Trp Ile Thr Phe Asp Leu
                245                 250                 255
Lys Asn Lys Glu Val Ser Val Lys Arg Val Thr Gln Asp Pro Lys Leu
            260                 265                 270
Gln Met Gly Lys Lys Leu Pro Leu His Leu Thr Leu Pro Gln Ala Leu
        275                 280                 285
Pro Gln Tyr Ala Gly Ser Gly Asn Leu Thr Leu Ala Leu Glu Ala Lys
    290                 295                 300
Thr Gly Lys Leu His Gln Glu Val Asn Leu Val Val Met Arg Ala Thr

305                 310                 315                 320
Gln Leu Gln Lys Asn Leu Thr Cys Glu Val Trp Gly Pro Thr Ser Pro
                325                 330                 335
Lys Leu Met Leu Ser Leu Lys Leu Glu Asn Lys Glu Ala Lys Val Ser
            340                 345                 350
Lys Arg Glu Lys Ala Val Trp Val Leu Asn Pro Glu Ala Gly Met Trp
        355                 360                 365
Gln Cys Leu Leu Ser Asp Ser Gly Gln Val Leu Leu Glu Ser Asn Ile
    370                 375                 380
Lys Val Leu Pro Thr Trp Ser Thr Pro Val Gln Pro Met Ala Leu Ile
385                 390                 395                 400
Val Leu Gly Gly Val Ala Gly Leu Leu Leu Phe Ile Gly Leu Gly Ile
                405                 410                 415
Phe Phe Cys Val Arg Cys Arg His Arg Arg Arg Gln Ala Glu Arg Met
            420                 425                 430
Ser Gln Ile Lys Arg Leu Leu Ser Glu Lys Lys Thr Cys Gln Cys Pro
        435                 440                 445
His Arg Phe Gln Lys Thr Cys Ser Pro Ile
    450                 455

<210> 16
<211> 235
<212> PRT
<213> Homo sapiens

<220>
<223> hCD8a

<400> 16

<210> 17
<211> 239
<212> PRT

<213> Artificial Sequence

<220>
<223> GFP

<400> 17
Met Val Ser Lys Gly Glu Glu Leu Phe Thr Gly Val Val Pro Ile Leu
1               5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Tyr Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235

<210> 18
<211> 2100
<212> DNA
<213> Artificial Sequence

<220>
<223> SB-NFATreg-GFP

<400> 18
cagttgaagt cggaagttta catacactta agttggagtc attaaaactc gtttttcaac      60

tactccacaa atttcttgtt aacaaacaat agttttggca agtcagttag gacatctact     120

ttgtgcatga cacaagtcat ttttccaaca attgtttaca gacagattat ttcacttata     180

attcactgta tcacaattcc agtgggtcag aagtttacat acactaagtt gactgtgcct     240

ttaaacagct tggaaaattc cagaaaatga tgtcatggct ttagaagctt ggcctttccg     300

gattgtgtct ccaccccaaa gaggaaaatt tgtttcatac agaaggcgtt cattgtaacc     360

ggattgtgtc tccaccccaa agaggaaaat ttgtttcata cagaaggcgt tcattgtaac     420

cggattgtgt ctccacccca aagaggaaaa tttgtttcat acagaaggcg ttcattgtaa     480

ccggattgtg tctccacccc aaagaggaaa atttgtttca tacagaaggc gttcattgta     540

accggattgt gtctccaccc caaagaggaa aatttgtttc atacagaagg cgttcattgt 600

aaccggattg tgtctccacc ccaaagagga aaatttgttt catacagaag gcgttcattg 660

taaccggtaa agtagactct ttgaaaatat gtgtaatatg taaaacatcg tgacaccccc 720

atattatttt tccagcatta acagtataaa ttgcctccca tgctgaagag ctgcctatca 780

cccttgctaa tcactcctca cagtgacctc aagtcctgca ggcaccggag aggcactggg 840

caggtaagta tcaaggttac aagacaggtt taaggagacc aatagaaact gggcttgtcg 900

agacagaacg cgtaattaat gcatgaagac tcttgcgttt ctgataggca cctattggtc 960

ttactgacat ccactttgcc tttctctcca caggtgtcca ctcccagcgg ccgcatggtg 1020

agcaagggcg aggagctgtt caccggggtg gtgcccatcc tggtcgagct ggacggcgac 1080

gtaaacggcc acaagttcag cgtgtccggc gagggcgagg gcgatgccac ctacggcaag 1140

ctgaccctga agttcatctg caccaccggc aagctgcccg tgccctggcc caccctcgtg 1200

accaccctga cctacggcgt gcagtgcttc agccgctacc ccgaccacat gaagcagcac 1260

gacttcttca agtccgccat gcccgaaggc tacgtccagg agcgcaccat cttcttcaag 1320

gacgacggca actacaagac ccgcgccgag gtgaagttcg agggcgacac cctggtgaac 1380

cgcatcgagc tgaagggcat cgacttcaag gaggacggca acatcctggg gcacaagctg 1440

gagtacaact acaacagcca caacgtctat atcatggccg acaagcagaa gaacggcatc 1500

aaggtgaact tcaagatccg ccacaacatc gaggacggca gcgtgcagct cgccgaccac 1560

taccagcaga acacccccat cggcgacggc cccgtgctgc tgcccgacaa ccactacctg 1620

agcacccagt ccgccctgag caaagacccc aacgagaagc gcgatcacat ggtcctgctg 1680

gagttcgtga ccgccgccgg gatcactctc ggcatggacg agctgtacaa gtaagaattc 1740

aataaaatat ctttattttc attacatctg tgtgttggtt ttttgtgtgt aattcggatc 1800

tagcttgtgg aaggctactc gaaatgtttg acccaagtta aacaatttaa aggcaatgct 1860

accaaatact aattgagtgt atgtaaactt ctgacccact gggaatgtga tgaaagaaat 1920

aaaagctgaa atgaatcatt ctctctacta ttattctgat atttcacatt cttaaaataa 1980

agtggtgatc ctaactgacc taagacaggg aatttttact aggattaaat gtcaggaatt 2040

gtgaaaaagt gagtttaaat gtatttggct aaggtgtatg taaacttccg acttcaactg 2100

<210> 19
<211> 34
<212> PRT
<213> Measles virus

<220>
<223> Hwt

<400> 19

Met Ser Pro Gln Arg Asp Arg Ile Asn Ala Phe Tyr Lys Asp Asn Pro
1               5                   10                  15
His Pro Lys Gly Ser Arg Ile Val Ile Asn Arg Glu His Leu Met Ile
            20                  25                  30
Asp Arg

<210> 20
<211> 20
<212> PRT
<213> Artificial Sequence

<220>
<223> Hd14

<400> 20
Met Pro His Pro Lys Gly Ser Arg Ile Val Ile Asn Arg Glu His Leu
1               5                   10                  15
Met Ile Asp Arg
            20

<210> 21
<211> 19
<212> PRT
<213> Artificial Sequence

<220>
<223> Hd15

<400> 21
Met His Pro Lys Gly Ser Arg Ile Val Ile Asn Arg Glu His Leu Met
1               5                   10                  15
Ile Asp Arg

<210> 22
<211> 18
<212> PRT
<213> Artificial Sequence

<220>
<223> Hd16

<400> 22
Met Pro Lys Gly Ser Arg Ile Val Ile Asn Arg Glu His Leu Met Ile
1               5                   10                  15
Asp Arg

<210> 23
<211> 17
<212> PRT
<213> Artificial Sequence

<220>
<223> Hd17

<400> 23
Met Lys Gly Ser Arg Ile Val Ile Asn Arg Glu His Leu Met Ile Asp
1               5                   10                  15
Arg

<210> 24
<211> 16
<212> PRT
<213> Artificial Sequence

<220>
<223> Hd18

<400> 24
Met Gly Ser Arg Ile Val Ile Asn Arg Glu His Leu Met Ile Asp Arg
1               5                   10                  15

<210> 25
<211> 15
<212> PRT
<213> Artificial Sequence

<220>
<223> Hd19

<400> 25
Met Ser Arg Ile Val Ile Asn Arg Glu His Leu Met Ile Asp Arg
1               5                   10                  15

<210> 26
<211> 14
<212> PRT
<213> Artificial Sequence

<220>
<223> Hd20

<400> 26
Met Arg Ile Val Ile Asn Arg Glu His Leu Met Ile Asp Arg
1               5                   10

<210> 27
<211> 13
<212> PRT
<213> Artificial Sequence

<220>
<223> Hd21

<400> 27
Met Ile Val Ile Asn Arg Glu His Leu Met Ile Asp Arg
1               5                   10

<210> 28
<211> 12
<212> PRT
<213> Artificial Sequence

<220>
<223> Hd22

<400> 28
Met Val Ile Asn Arg Glu His Leu Met Ile Asp Arg
1               5                   10

<210> 29

<211> 11
<212> PRT
<213> Artificial Sequence

<220>
<223> Hd23

<400> 29
Met Ile Asn Arg Glu His Leu Met Ile Asp Arg
1               5                   10

<210> 30
<211> 10
<212> PRT
<213> Artificial Sequence

<220>
<223> Hd24

<400> 30
Met Asn Arg Glu His Leu Met Ile Asp Arg
1               5                   10

<210> 31
<211> 14
<212> PRT
<213> Artificial Sequence

<220>
<223> Hd21+1A

<400> 31
Met Ala Ile Val Ile Asn Arg Glu His Leu Met Ile Asp Arg
1               5                   10

<210> 32
<211> 14
<212> PRT
<213> Artificial Sequence

<220>
<223> Hd24+4A

<400> 32
Met Ala Ala Ala Ala Asn Arg Glu His Leu Met Ile Asp Arg
1               5                   10

<210> 33
<211> 14
<212> PRT
<213> Artificial Sequence

<220>
<223> Hd26+6A

<400> 33
Met Ala Ala Ala Ala Ala Ala Glu His Leu Met Ile Asp Arg
1               5                   10

<210> 34
<211> 14
<212> PRT
<213> Artificial Sequence

<220>
<223> Hd30+10A

<400> 34
Met Ala Ala Ala Ala Ala Ala Ala Ala Ala Ala Ile Asp Arg
1               5                   10

<210> 35
<211> 35
<212> PRT
<213> Measles virus

<220>
<223> Fwt

<400> 35
Cys Cys Arg Gly Arg Cys Asn Lys Lys Gly Glu Gln Val Gly Met Ser
1               5                   10                  15
Arg Pro Gly Leu Lys Pro Asp Leu Thr Gly Thr Ser Lys Ser Tyr Val
            20                  25                  30
Arg Ser Leu
        35

<210> 36
<211> 11
<212> PRT
<213> Artificial Sequence

<220>
<223> Fd24

<400> 36
Cys Cys Arg Gly Arg Cys Asn Lys Lys Gly Glu
1               5                   10

<210> 37
<211> 5
<212> PRT
<213> Artificial Sequence

<220>
<223> Fd30

<400> 37
Cys Cys Arg Gly Arg
1               5